# EUROPEAN PATENT APPLICATION

(11) **EP 4 783 191 A1**
(43) Date of publication of application: **29.07.2026**
(21) Application number: 26153803.7
(22) Date of filing: 23.01.2026
(51) Int. Cl.: G16H 40/67, G16H 20/30, G16H 40/63, G16H 50/20

(54) **ARTIFICIAL INTELLIGENCE (AI) AGENT SYSTEMS AND METHODS FOR DYNAMICALLY GENERATING CONTENT REGARDING ORAL ACTIVITY**

(30) Priority: 24.01.2025 US 202563749426 P; 09.05.2025 US 202563803383 P
(71) Applicant: The Procter & Gamble Company, Cincinnati, OH 45202 (US)
(72) Inventor: SHERMAN, Faiz Feisal, Cincinnati, 45202 (US); KHAN, Meraj, Cincinnati, 45202 (US); MAO, Xiaole, Cincinnati, 45202 (US)
(74) Representative: Patent Boutique LLP

(57) **Abstract**

Artificial intelligence (AI) agent systems and methods are described for dynamically generating content regarding oral activity. A query comprising natural language including natural language tokens defining oral activity of a user is received and used as input to an AI agent program to dynamically determine executable code programmed to address the oral activity. Content as output for responding to the query is dynamically generated based on execution of the executable code. The content is transmitted to a computing device of the user for output by the computing device regarding the oral activity.

## Description

### FIELD

The present disclosure generally relates to artificial intelligence (AI) agent systems and methods, and, more particularly, to AI agent systems and methods configured to dynamically generate content regarding oral activity, which may include, for example, dynamically determining executable code for execution that causes dynamic generation of content as output for responding to a query having natural language tokens and/or video images regarding oral activity.

### BACKGROUND

Oral care, though often perceived as straightforward, is a multifaceted task that presents significant challenges for many individuals. While widely recommended routines-such as brushing, flossing, and rinsing with mouthwash-are commonly followed, these efforts frequently fall short of achieving desired results.

From a technical standpoint, effective oral care necessitates a foundational understanding of the biological processes involved, including the formation and behavior of oral bacterial biofilm (plaque) and the structure and health of oral tissues like teeth and gums. It also requires familiarity with the mechanical, biological, and chemical properties of oral care products. Unfortunately, many consumers lack the oral health education needed to make well-informed choices tailored to their specific needs and challenges.

A tailored oral care approach must consider individual behaviors, such as brushing technique, the pressure applied during brushing, and the frequency of these activities. Additionally, unique factors like pre-existing oral health issues, dietary habits, and the anatomical differences in oral biology must be accounted for. While professional guidance during dental check-ups can offer personalized insights, these visits are typically limited to twice a year for most individuals. This infrequency constrains the availability of ongoing, personalized support, leaving consumers without the consistent feedback necessary to refine their daily practices.

Further, current electronic devices are currently limited. That is, although recent innovations, such as sensor-based technology, can be used to measure user activity, consumers struggle to leverage sensor data effectively. The lack of oral health expertise among users hampers their ability to interpret the data, relate it to their unique oral health conditions, and implement actionable, personalized improvements.

Additionally, digital tools such as brushing guidance apps face limitations in addressing the diverse needs of individual users. Many of these tools rely on rigid, hard-coded functionalities that do not fully account for the variability in oral health challenges and behaviors.

This problem highlights the need for more adaptive, user-centered technologies capable of addressing the specific requirements of each consumer. For the foregoing reasons, there is a need for AI agent systems and methods configured to dynamically generate content regarding oral activity, which may include, for example, dynamically determining executable code for execution that causes dynamic generation of content as output for responding to a query having natural language tokens and/or video images regarding oral activity.

### SUMMARY

The present disclosure is an oral care agentic system, described herein as artificial intelligence (AI) agent systems and methods for dynamically generating content regarding oral activity. In various aspects, the AI agent systems and methods implement large AI foundational models, such as large language models (LLMs) having millions or billions of parameters and trained to receive user queries and generate natural language tokens. By analyzing a given query and/or its respective natural language tokens, the AI agent systems and methods describe herein can accurately determine intention(s) behind a user's query inquiries by dynamically determining (e.g., identifying or generating) executable code by an appropriate AI agent program to address the user's specific oral activity. In some aspects, a user's given query may be routed to an AI agent router configured to receive the query and select from one or more AI agent programs to address the user's specific oral activity as determined based on the query and/or its natural language tokens.

In some aspects, the techniques described herein relate to an artificial intelligence (AI) agent system configured to dynamically generate content regarding oral activity, the AI agent system including: a computer memory communicatively coupled to one or more processors, an AI agent program including computing instructions stored on the computer memory, and wherein the AI agent program is accessible by the one or more processors; wherein one or more oral activity files are stored on the computer memory, each oral activity file including data related to oral activity of respective users during respective oral activity sessions, computing instructions stored on the computer memory that, when executed by the one or more processors, cause the one or more processors to: receive a query including natural language including natural language tokens defining oral activity of a user, dynamically determine, by the AI agent program using the natural language of the query as input, executable code programmed to address the oral activity, dynamically generate, based on execution of the executable code, content as output for responding to the query, and transmit the content to a computing device of the user for output by the computing device regarding the oral activity.

In some aspects, the techniques described herein relate to an AI agent system, wherein the natural language is provided as text input or in audible voice input, and wherein the natural language tokens include text-based tokens or audio tokens.

In some aspects, the techniques described herein relate to an AI agent system, wherein the natural language tokens include units of computation.

In some aspects, the techniques described herein relate to an AI agent system, wherein the natural language tokens include bytes or patches.

In some aspects, the techniques described herein relate to an AI agent system, wherein the content as dynamically generated based on the execution of the executable code includes one of: (a) a chart; (b) a graphic; (c) a textual output; and (d) audible output, and (e) video.

In some aspects, the techniques described herein relate to an AI agent system, wherein the computing instructions stored on the computer memory, when executed by the one or more processors, further cause the one or more processors to: prior to dynamically generating the content as output, generating intermediate data, executable code, or graphics, and wherein the content is generated based on the intermediate data, executable code, or graphics.

In some aspects, the techniques described herein relate to an AI agent system, wherein the computing device includes an oral care implement, and wherein the content includes computing instructions configured to change one or more settings of the oral care implement.

In some aspects, the techniques described herein relate to an AI agent system further including an AI agent router executing on one or more processors, and wherein the computing instructions, when executed by the one or more processors, further cause the one or more processors to: analyze, by the AI agent router, the natural language tokens to select the AI agent program from one or more AI agent programs configured to provide output for the query, and route, by the AI agent router, the query to the AI agent program as selected.

In some aspects, the techniques described herein relate to an AI agent system, wherein a user-specific tooth brushing behavior file of the user that is stored on the computer memory and defines user-specific tooth brushing metrics of the user when the user brushed during respective user tooth brushing sessions, the user-specific tooth brushing metrics further defining a plurality of mouth zones, wherein the query includes the natural language tokens related to oral activity of the user, wherein the AI agent program includes a brushing AI agent program, and wherein the computing instructions, that when executed by the one or more processors, further cause the one or more processors to: access the user-specific tooth brushing metrics, invoke the brushing AI agent program to dynamically determine the executable code, and analyze the user-specific tooth brushing behavior file to determine at least one of: (a) brushing times of the user across the plurality of mouth zones; (b) brushing pressure at any one or more of the plurality of mouth zones; (c) brushing movement patterns at any one or more of the plurality of mouth zones; and/or (d) brushing dwell times at any one or more of the plurality of mouth zones.

In some aspects, the techniques described herein relate to an AI agent system, wherein the user-specific tooth brushing behavior file includes headers or metadata identifying each of the plurality of mouth zones, wherein a pre-engineered prompt defines descriptions of the headers or metadata, wherein the AI agent program invokes a generative AI model that associates, based on the headers or metadata, the descriptions to the user-specific tooth brushing metrics, wherein the generative AI model inputs the pre-engineered prompt and the user-specific tooth brushing behavior file and outputs at least one of the executable code or the content, and wherein the executable code or the content include output defined by the descriptions of the pre-engineered prompt.

In some aspects, the techniques described herein relate to an AI agent system, wherein the content generated based on execution of the executable code includes a chart graphically depicting the user's brushing time for each of the plurality of mouth zones.

In some aspects, the techniques described herein relate to an AI agent system, wherein the chart graphically depicts a line representing an ideal duration for brushing across each of the plurality of mouth zones.

In some aspects, the techniques described herein relate to an AI agent system, wherein the content includes user-specific coaching content including at least one of: textual output, audible output, or video output created to improve the user's user-specific tooth brushing behavior for one or more of the plurality of mouth zones.

In some aspects, the techniques described herein relate to an AI agent system, wherein the AI agent program includes or is configured to access a generative AI model for dynamically generating or determining at least one of the executable code or the content.

In some aspects, the techniques described herein relate to an AI agent system, wherein an AI agent program provides as input a pre-engineered prompt to the generative AI model, and wherein the generative AI model generates or determines at least one of the executable code or the content based on the pre-engineered prompt.

In some aspects, the techniques described herein relate to an AI agent system, wherein the query includes one of: (a) a user query of the user; or (b) a system query invoked by the one or more processors.

In some aspects, the techniques described herein relate to an AI agent system, wherein the system query is a predefined query stored in the computer memory.

In some aspects, the techniques described herein relate to an AI agent system, wherein the system query is linked to a graphic selectable by the user from a graphic user interface (GUI) displayed on a display screen, and wherein when the graphic is selected, the one or more processors invoke the system query.

In some aspects, the techniques described herein relate to an AI agent system, wherein the system query is invoked periodically.

In some aspects, the techniques described herein relate to an AI agent system, wherein the computing instructions stored on the computer memory, when executed by the one or more processors, further cause the one or more processors to: receive a video depicting brushing activity during a brushing session and including multimedia tokens, wherein the query including the natural language includes a query about the video, analyze, by the executable code, the multimedia tokens to address the oral activity, wherein the content as output for responding to query is based at least in part on the brushing activity depicted in the video.

In some aspects, the techniques described herein relate to an AI agent system, wherein the video includes image frames of the user, and wherein the video is captured by a second user.

In some aspects, the techniques described herein relate to an AI agent system, wherein a reward is displayed to the user when the user achieves an oral activity goal.

In some aspects, the techniques described herein relate to an AI agent system, wherein dynamically determining the executable code includes one of: (a) dynamically identifying preexisting executable code stored on the computer memory and programmed to address a specific query; or (b) dynamically generating new executable code created to address the oral activity.

In some aspects, the techniques described herein relate to an artificial intelligence (AI) agent method for dynamically generating content regarding oral activity, the AI agent method including: receiving, by one or more processors, a query including natural language including natural language tokens defining oral activity of a user, dynamically determining, by an AI agent program executing on the one or more processors and using the natural language of the query as input, executable code programmed to address the oral activity, wherein the AI agent program includes computing instructions stored on a computer memory, and wherein one or more oral activity files are stored on the computer memory, each oral activity file including data related to oral activity of respective users during respective oral activity sessions, dynamically generating, based on execution of the executable code by the one or more processors, content as output for responding to the query, and transmitting, by the one or more processors, the content to a computing device of the user for output by the computing device regarding the oral activity.

In some aspects, the techniques described herein relate to an AI agent method, wherein the natural language is provided as text input or in audible voice input, and wherein the natural language tokens include text-based tokens or audio tokens.

In some aspects, the techniques described herein relate to an AI agent method, wherein the natural language tokens include units of computation.

In some aspects, the techniques described herein relate to an AI agent method, wherein the natural language tokens include bytes or patches.

In some aspects, the techniques described herein relate to an AI agent method, wherein the content as dynamically generated based on the execution of the executable code includes one of: (a) a chart; (b) a graphic; (c) a textual output; and (d) audible output, and (e) video.

In some aspects, the techniques described herein relate to an AI agent method further including: prior to dynamically generating the content as output, generating intermediate data, executable code, or graphics, and wherein the content is generated based on the intermediate data, executable code, or graphics.

In some aspects, the techniques described herein relate to an AI agent method, wherein the computing device includes an oral care implement, and wherein the content includes computing instructions configured to change one or more settings of the oral care implement.

In some aspects, the techniques described herein relate to an AI agent method further including: analyzing, by an AI agent router executing on the one or more processors, the natural language tokens to select the AI agent program from one or more AI agent programs configured to provide output for the query, routing, by the AI agent router, the query to the AI agent program as selected.

In some aspects, the techniques described herein relate to an AI agent method, wherein a user-specific tooth brushing behavior file of the user that is stored on the computer memory and defines user-specific tooth brushing metrics of the user when the user brushed during respective user tooth brushing sessions, the user-specific tooth brushing metrics further defining a plurality of mouth zones, wherein the query includes the natural language tokens related to oral activity of the user, wherein the AI agent program includes a brushing AI agent program, and wherein the AI agent method further includes: accessing the user-specific tooth brushing metrics, invoking the brushing AI agent program to dynamically determine the executable code, and analyzing the user-specific tooth brushing behavior file to determine at least one of: (a) brushing times of the user across the plurality of mouth zones; (b) brushing pressure at any one or more of the plurality of mouth zones; (c) brushing movement patterns at any one or more of the plurality of mouth zones; and/or (d) brushing dwell times at any one or more of the plurality of mouth zones.

In some aspects, the techniques described herein relate to an AI agent method, wherein the user-specific tooth brushing behavior file includes headers or metadata identifying each of the plurality of mouth zones, wherein a pre-engineered prompt defines descriptions of the headers or metadata, wherein the AI agent program invokes a generative AI model that associates, based on the headers or metadata, the descriptions to the user-specific tooth brushing metrics, wherein the generative AI model inputs the pre-engineered prompt and the user-specific tooth brushing behavior file and outputs at least one of the executable code or the content, and wherein the executable code or the content include output defined by the descriptions of the pre-engineered prompt.

In some aspects, the techniques described herein relate to an AI agent method, wherein the content generated based on execution of the executable code includes a chart graphically depicting the user's brushing time for each of the plurality of mouth zones.

In some aspects, the techniques described herein relate to an AI agent method, wherein the chart graphically depicts a line representing an ideal duration for brushing across each of the plurality of mouth zones.

In some aspects, the techniques described herein relate to an AI agent method, wherein the content includes user-specific coaching content including at least one of: textual output, audible output, or video output created to improve the user's user-specific tooth brushing behavior for one or more of the plurality of mouth zones.

In some aspects, the techniques described herein relate to an AI agent method, wherein the AI agent program includes or is configured to access a generative AI model for dynamically generating or determining at least one of the executable code or the content.

In some aspects, the techniques described herein relate to an AI agent method, wherein an AI agent program provides as input a pre-engineered prompt to the generative AI model, and wherein the generative AI model generates or determines at least one of the executable code or the content based on the pre-engineered prompt.

In some aspects, the techniques described herein relate to an AI agent method, wherein the query includes one of: (a) a user query of the user; or (b) a system query invoked by the one or more processors.

In some aspects, the techniques described herein relate to an AI agent method, wherein the system query is a predefined query stored in the computer memory.

In some aspects, the techniques described herein relate to an AI agent method, wherein the system query is linked to a graphic selectable by the user from a graphic user interface (GUI) displayed on a display screen, and wherein when the graphic is selected, the one or more processors invoke the system query.

In some aspects, the techniques described herein relate to an AI agent method, wherein the system query is invoked periodically.

In some aspects, the techniques described herein relate to an AI agent method further including: receiving a video depicting brushing activity during a brushing session and including multimedia tokens, wherein the query including the natural language includes a query about the video, analyzing, by the executable code, the multimedia tokens to address the oral activity, wherein the content as output for responding to query is based at least in part on the brushing activity depicted in the video.

In some aspects, the techniques described herein relate to an AI agent method, wherein the video includes image frames of the user, and wherein the video is captured by a second user.

In some aspects, the techniques described herein relate to an AI agent method, wherein a reward is displayed to the user when the user achieves an oral activity goal.

In some aspects, the techniques described herein relate to an AI agent method, wherein dynamically determining the executable code includes one of: (a) dynamically identifying preexisting executable code stored on the computer memory and programmed to address a specific query; or (b) dynamically generating new executable code created to address the oral activity.

In some aspects, the techniques described herein relate to a tangible, non-transitory computer-readable medium storing instructions for dynamically generating content regarding oral activity, that when executed by one or more processors cause the one or more processors to: receive, by one or more processors, a query including natural language including natural language tokens defining oral activity of a user, dynamically determine, by an AI agent program executing on the one or more processors and using the natural language of the query as input, executable code programmed to address the oral activity, wherein the AI agent program includes computing instructions stored on a computer memory, and wherein one or more oral activity files are stored on the computer memory, each oral activity file including data related to oral activity of respective users during respective oral activity sessions, dynamically generate, based on execution of the executable code by the one or more processors, content as output for responding to the query, and transmit, by the one or more processors, the content to a computing device of the user for output by the computing device regarding the oral activity.

In accordance with the above, and with the disclosure herein, the present disclosure includes improvements in computer functionality or in improvements to other technologies at least because the disclosure describes that, *e.g.,* a server or otherwise computing device (*e.g.*, a mobile device), is improved by the use of computing instructions and AI agent program(s), which are lightweight programs configured to access a generative AI model for creating user-specific content based on natural language token analysis. That is, the present disclosure describes improvements in the functioning of the computer itself or "any other technology or technical field" because an server or user computing device is enhanced with AI agent program that can interface with or otherwise invoke an AI model (*e.g.*, a generative AI model), which can generate content without the need for memory intensive program code and/or data stored in a database (*e.g*., such as oral care data) in order to accurately generate dynamic content (*e.g*., in real-time) to address the user's specific oral activity. This improves over the prior art at least because existing systems lack such functionality and are simply not capable of accurately analyzing user-specific queries to dynamically generate content, *e.g*., in real-time. Instead, current systems require computing and memory intensive systems to store images, data, and other oral care information, that further require intensive compute cycles and memory access from manual manipulation of such images, data, and oral care information in order to produce similar content as the AI agent systems and methods for dynamically generating content regarding oral activity, as described herein.

For similar reasons, the present disclosure relates to improvements to other technologies or technical fields at least because the present disclosure describes or introduces improvements to computing devices (*e.g.*, a server or mobile computing device), whereby a generative AI model can be installed on a low memory storage device (*e.g*., RAM or ROM) and accessed for dynamically generating the content herein. Additionally, or alternatively, a generative AI model is accessible via a computer network such that there is no need to install any generative AI model on the underlying computing device (*e.g*., a server or mobile device). Use of a zero-installed (not installed) or a low memory generative AI model makes the underlying computing devices which access the generative AI models more efficient and improved because they can operate by using fewer compute cycles and memory. Further, the generative AI models may be configured, adjusted, adapted, and/or otherwise updated to use reduced memory. For example, in some embodiments, fewer machine resources (*e.g*., processing cycles or memory storage) may be used by decreasing computational resources by decreasing machine-learning network architecture needed to analyze user queries, including by reducing parameters of the generative AI model (*e.g.*, and therefore its required computer memory). Such a reduction frees up the computational resources of an underlying computing system, thereby improving it by making it more efficient.

In addition, the present disclosure includes specific features other than what is well-understood, routine, conventional activity in the field, or adding unconventional steps that confine the claim to a particular useful application, *e.g*., AI agent systems and methods configured to dynamically generate content regarding oral activity, which may include, for example, dynamically determining executable code for execution for dynamically generating the content as output for responding to a query regarding oral activity.

Advantages will become more apparent to those of ordinary skill in the art from the following description of the preferred embodiments which have been shown and described by way of illustration. As will be realized, the present embodiments may be capable of other and different embodiments, and their details are capable of modification in various respects. Accordingly, the drawings and description are to be regarded as illustrative in nature and not as restrictive.

### BRIEF DESCRIPTION OF THE DRAWINGS

The Figures described below depict various aspects of the system and methods disclosed therein. It should be understood that each Figure depicts an embodiment of a particular aspect of the disclosed system and methods, and that each of the Figures is intended to accord with a possible embodiment thereof. Further, wherever possible, the following description refers to the reference numerals included in the following Figures, in which features depicted in multiple Figures are designated with consistent reference numerals.

There are shown in the drawings arrangements which are presently discussed, it being understood, however, that the present embodiments are not limited to the precise arrangements and instrumentalities shown, wherein:

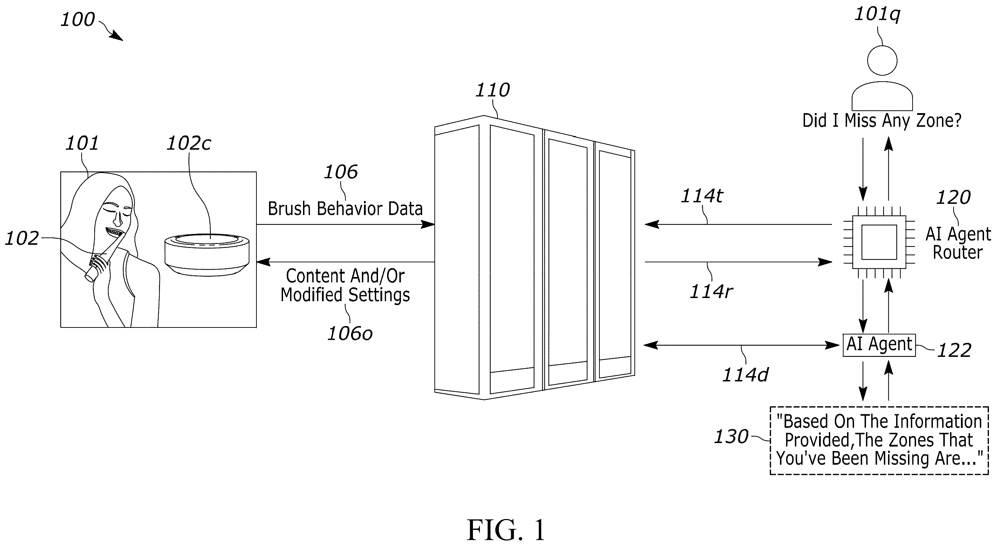

FIG. 1 illustrates an example artificial intelligence (AI) agent system configured to dynamically generate content regarding oral activity, in accordance with various embodiments disclosed herein.

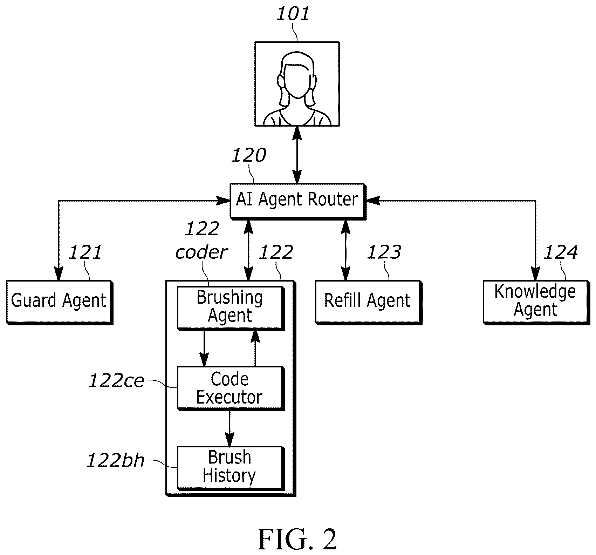

FIG. 2 illustrates an example an AI agent router configured to select an AI agent program from one or more AI agent programs based on natural language tokens of a query and configured to route the query to the AI agent program for providing output for the query, in accordance with various embodiments disclosed herein.

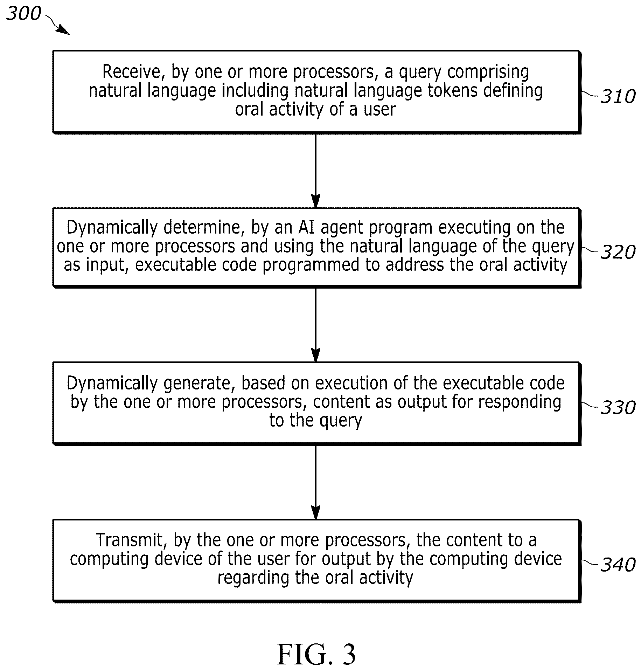

FIG. 3 illustrates an example AI agent method for dynamically generating content regarding oral activity, in accordance with various embodiments disclosed herein.

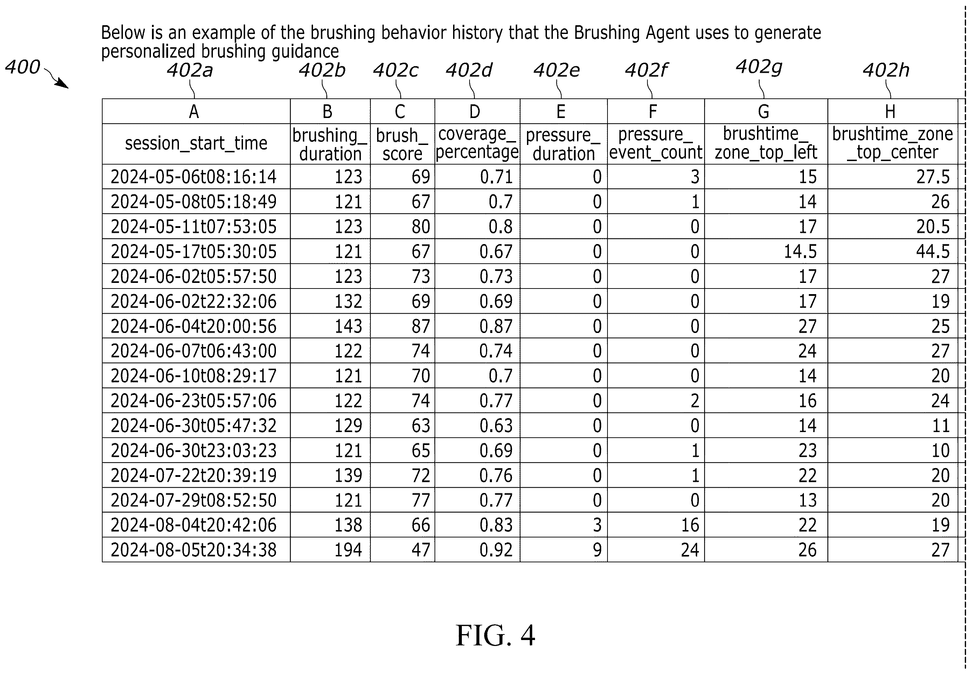

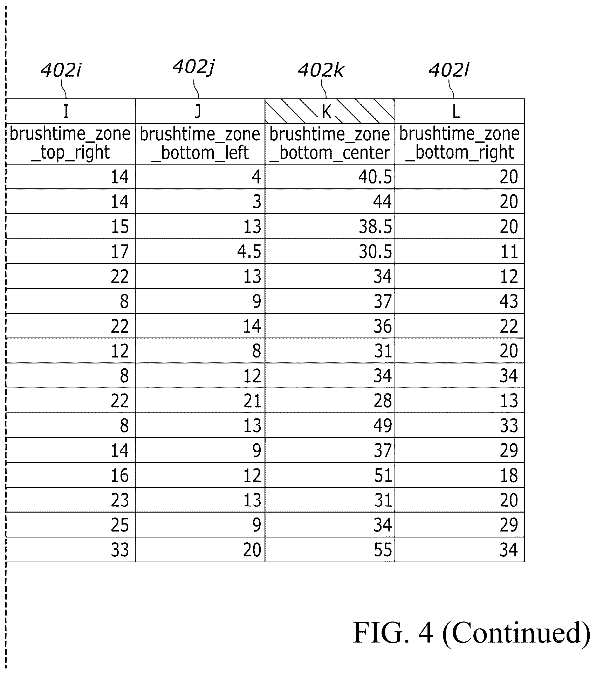

FIG. 4 illustrates an example user-specific tooth brushing behavior file or otherwise the oral activity file of a user, in accordance with various embodiments disclosed herein.

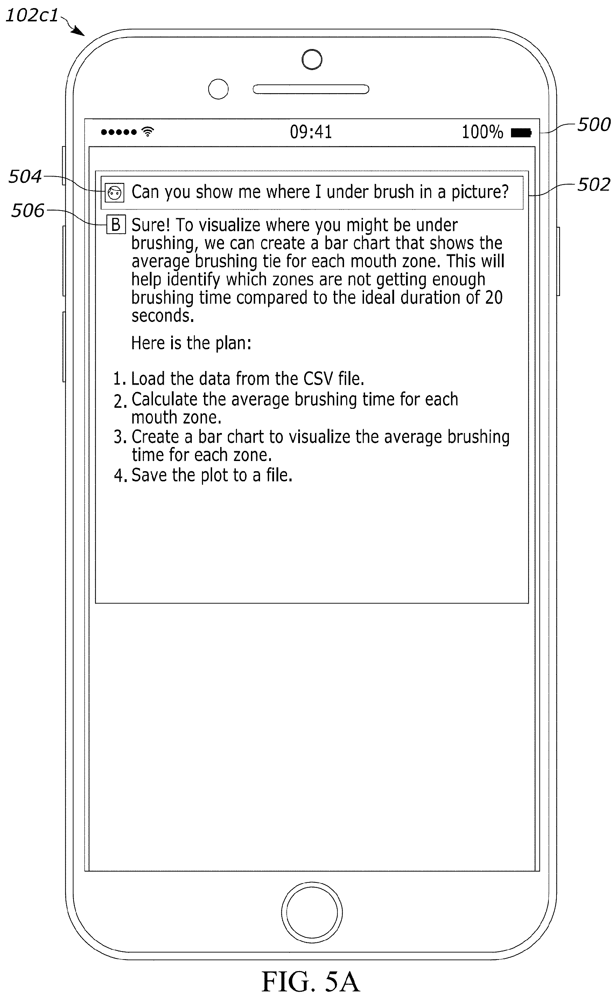

FIG. 5A illustrates an example user interface as rendered on a display screen of a computing device of a user and depicting an example query comprising natural language regarding a request visualize under brushing zones of the user (*e.g.*, as identifiable by the user's user-specific tooth brushing behavior file as shown for FIG. 4 or otherwise the user's oral activity file), in accordance with various embodiments disclosed herein.

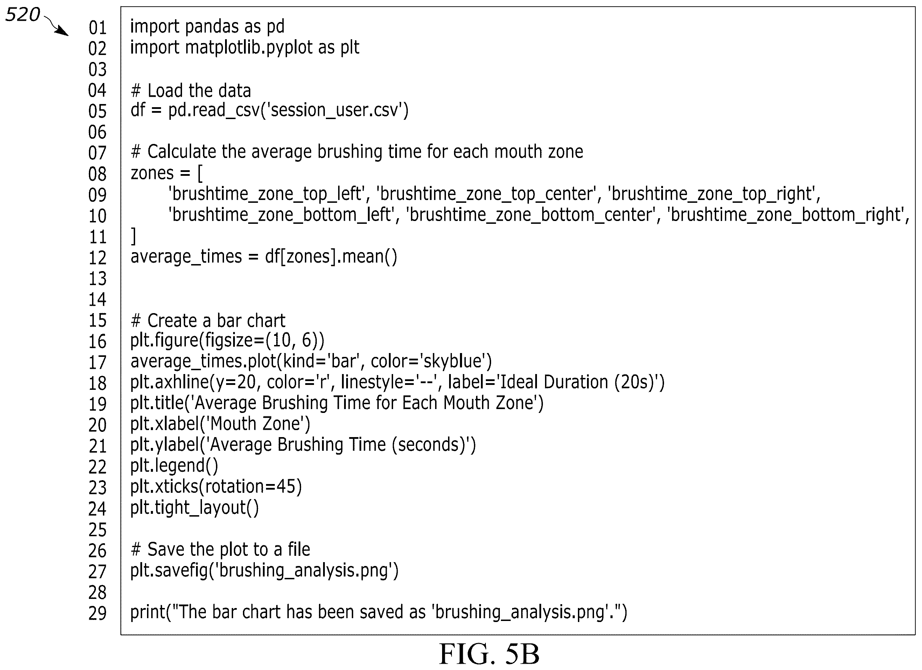

FIG. 5B illustrates an example of executable code comprising computing instructions as dynamically determined by the AI agent using the natural language of the query of FIG. 5A as input and programmed to address the oral activity as identified for FIG. 5A, in accordance with various embodiments disclosed herein.

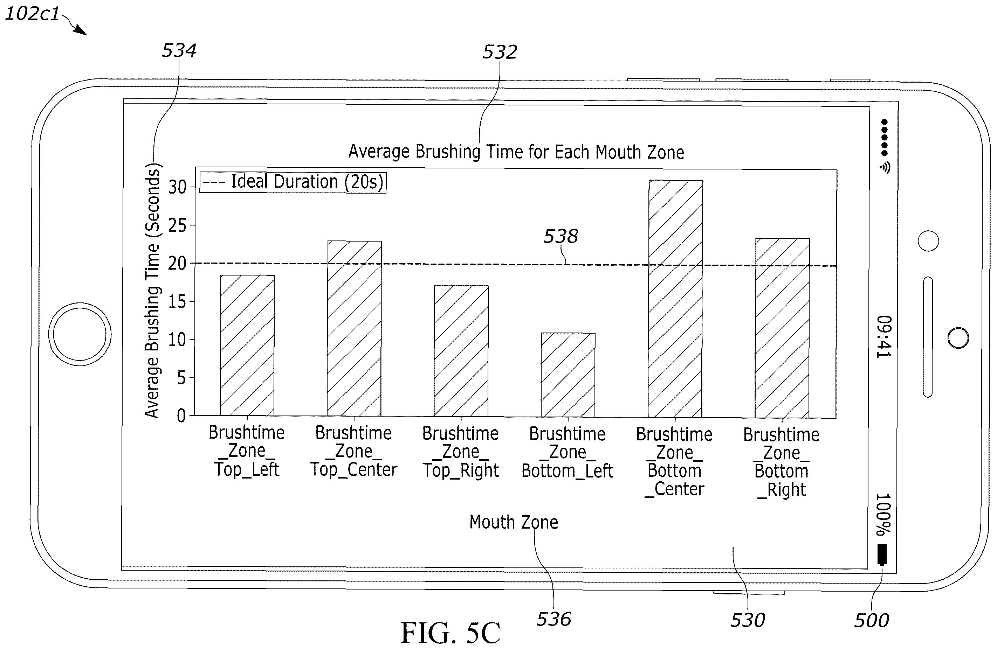

FIG. 5C illustrates an example content for responding to the query of FIG. 5A, as dynamically generated based on the executable code as described for FIG. 5B, and as transmitted to and displayed on the computing device of the user of FIG. 5A, in accordance with various embodiments disclosed herein.

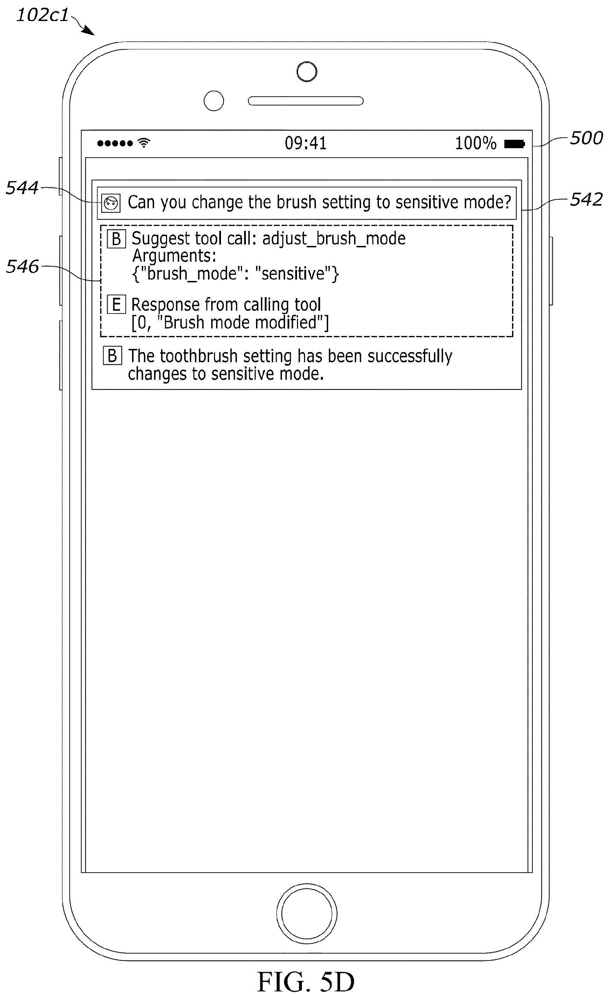

FIG. 5D illustrates a further example user interface as rendered on a display screen of a computing device of the user of FIG. 5A depicting an example query comprising natural language regarding a request to change the brush setting, in accordance with various embodiments disclosed herein.

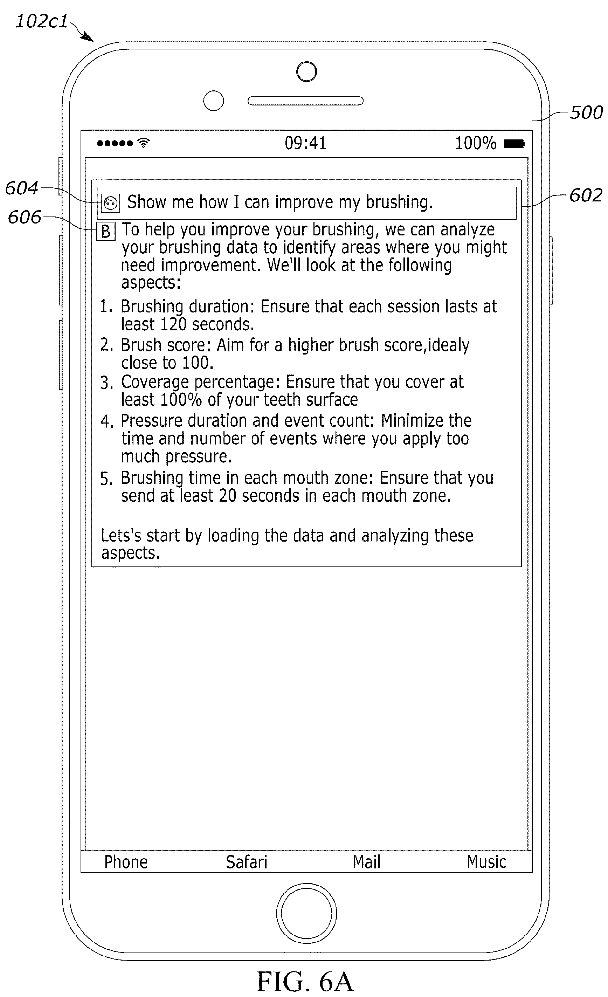

FIG. 6A illustrates an example user interface as rendered on a display screen of a computing device of a user and depicting an example query comprising natural language regarding a request to improve brushing (*e.g*., as identifiable by the user's user-specific tooth brushing behavior file as shown for FIG. 4 or otherwise the user's oral activity file), in accordance with various embodiments disclosed herein.

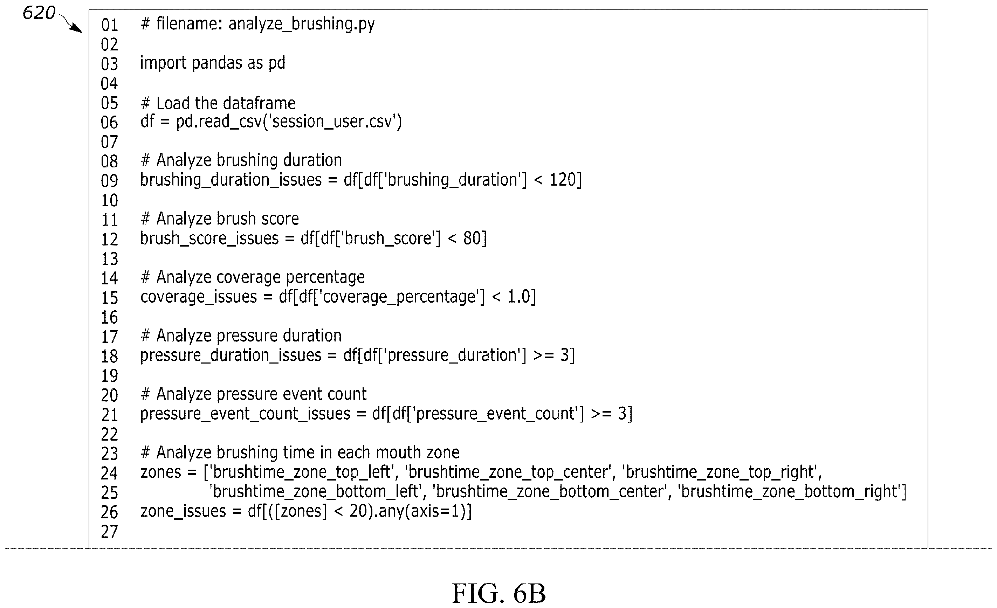

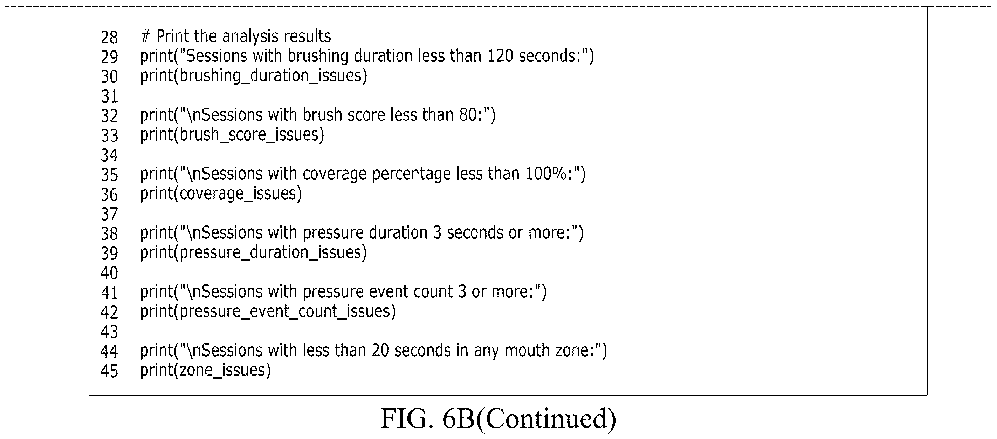

FIG. 6B illustrates an example of executable code comprising computing instructions as dynamically determined by the AI agent using the natural language of the query of FIG. 6A as input and programmed to address the oral activity as identified for FIG. 6A, in accordance with various embodiments disclosed herein.

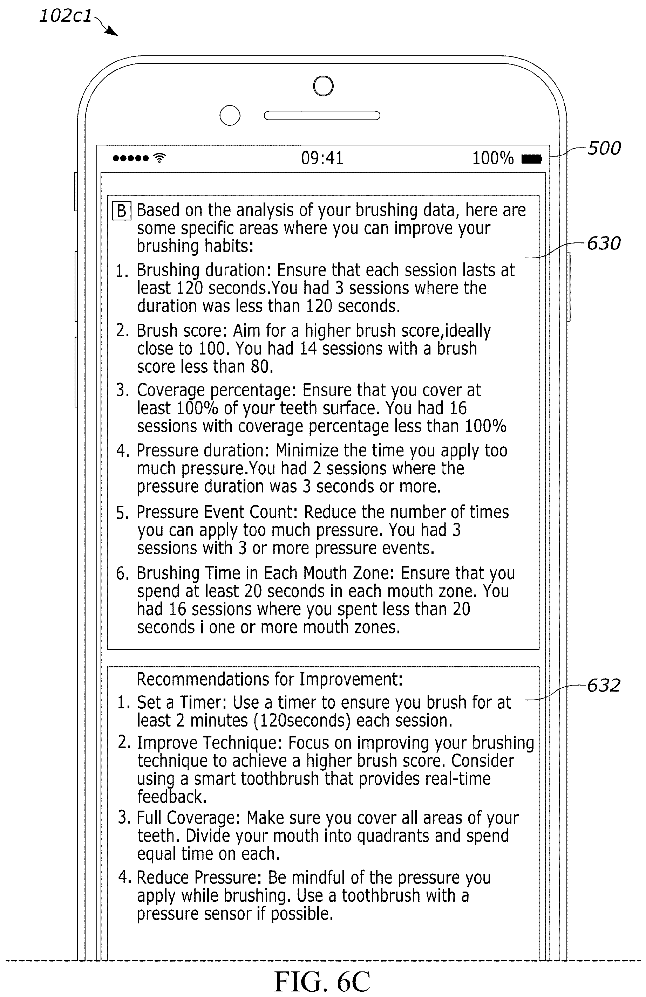

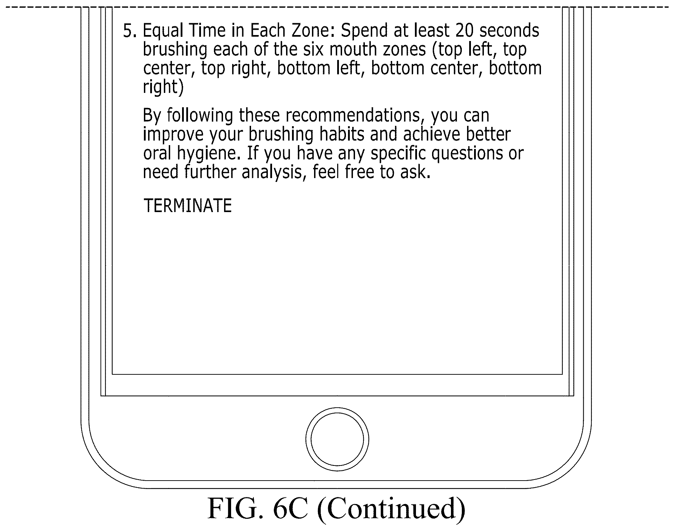

FIG. 6C illustrates an example content for responding to the query of FIG. 6A, as dynamically generated based on the executable code as described for FIG. 6B, and as transmitted to and displayed on the computing device of the user of FIG. 6A, in accordance with various embodiments disclosed herein.

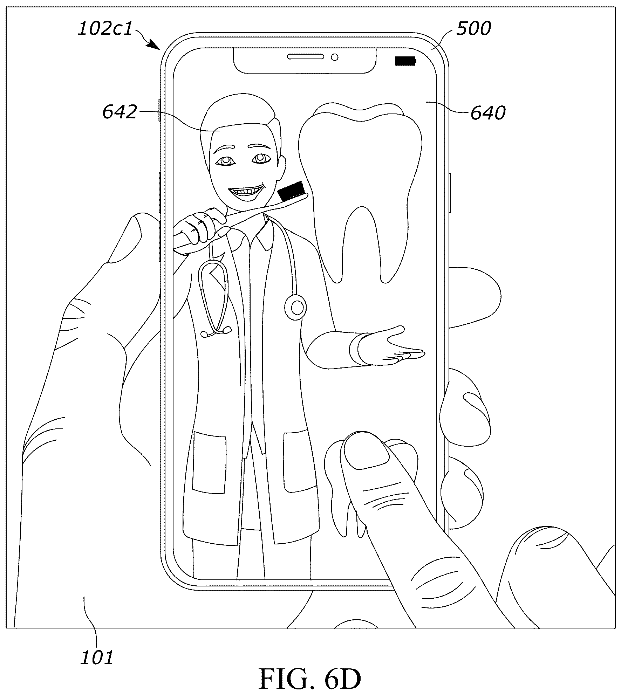

FIG. 6D illustrates a further example content for responding to the query of FIG. 6A, as dynamically generated based on the executable code as described for FIG. 6B, and as transmitted to and displayed on the computing device of the user of FIG. 6A, in accordance with various embodiments disclosed herein.

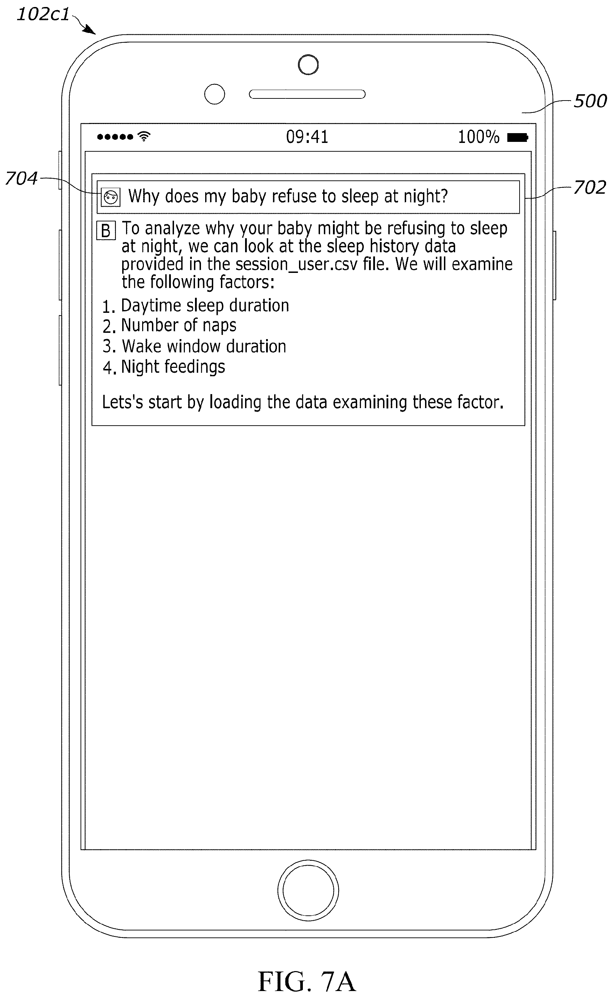

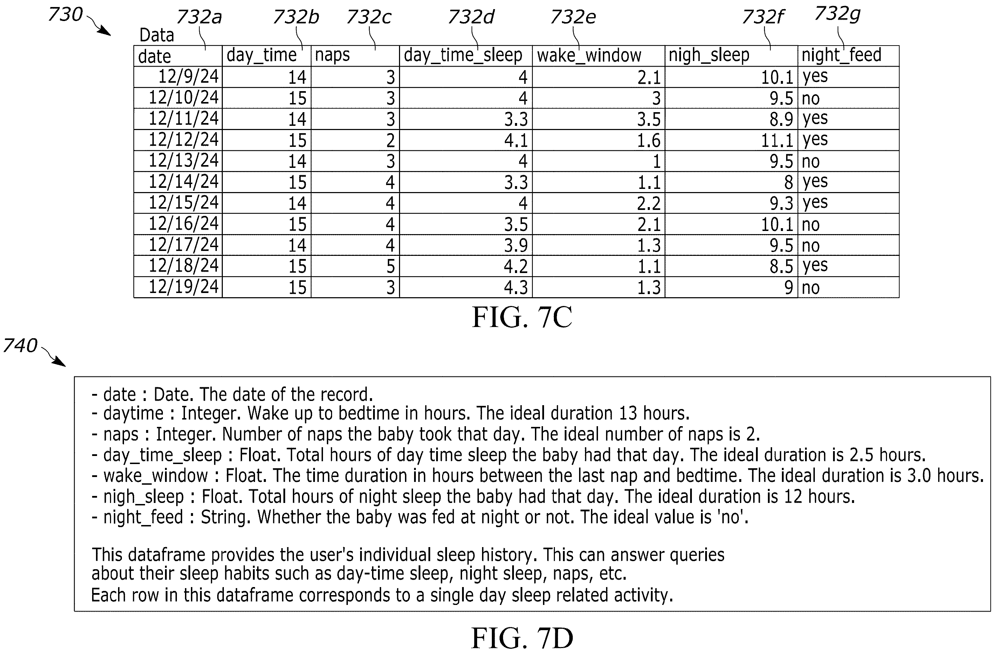

FIG. 7A illustrates an example user interface as rendered on a display screen of a computing device of a user and depicting an example query comprising natural language regarding a request regarding a baby care use case (*e.g*., as identifiable by a user's user-specific baby care file as shown for FIG. 7C or otherwise herein), and in accordance with various embodiments disclosed herein.

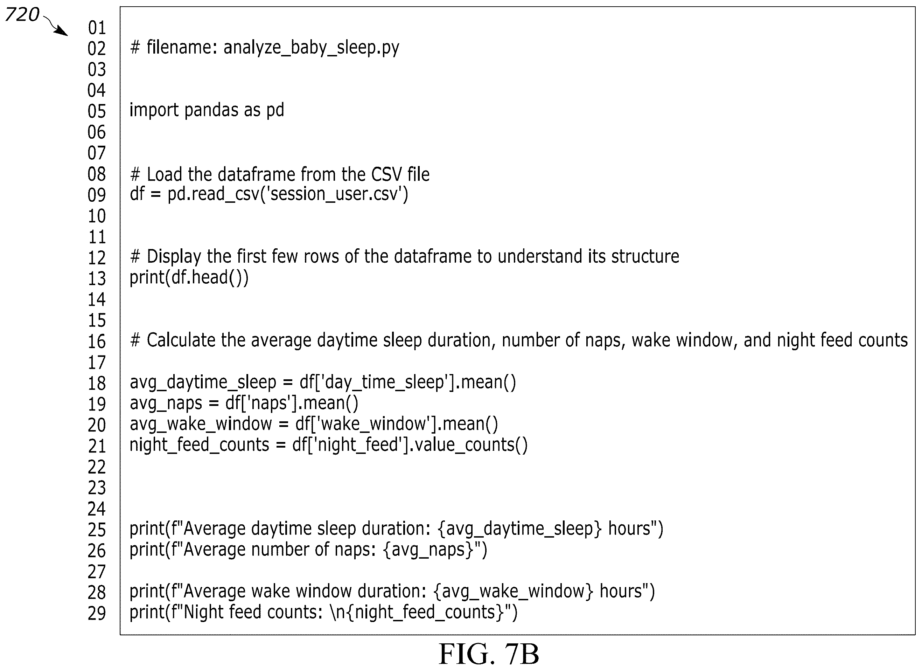

FIG. 7B illustrates an example of executable code comprising computing instructions as dynamically determined by an AI agent using the natural language of the query of FIG. 7A as input and programmed to address the baby care use case as identified for FIG. 7A, in accordance with various embodiments disclosed herein.
FIG. 7C illustrates an example of a user-specific baby care file as accessible by executable code as described by FIG. 7B, in accordance with various embodiments disclosed herein.
FIG. 7D illustrates an example of a pre-engineered prompt, or portion thereof, that defines descriptions of headers or metadata, including each of the headers or metadata of the user-specific baby care file as described for FIG. 7C, where the pre-engineered prompt (or portion thereof) of FIG. 7C can be provided to an AI agent program that can provide the pre-engineered prompt (or portion thereof) to a generative AI model as described herein, in accordance with various embodiments disclosed herein.

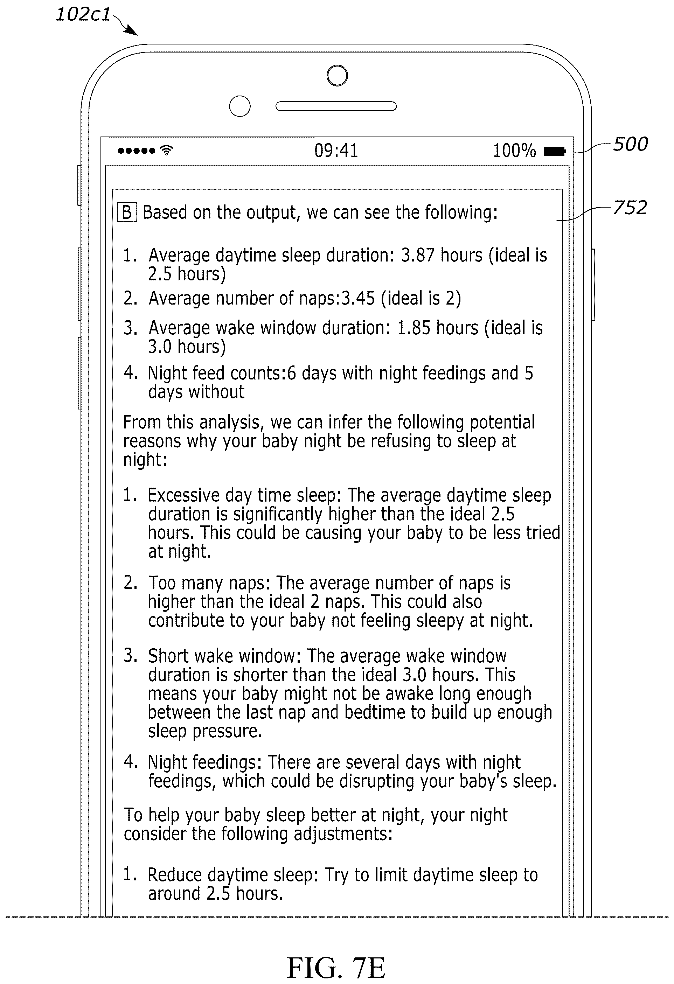

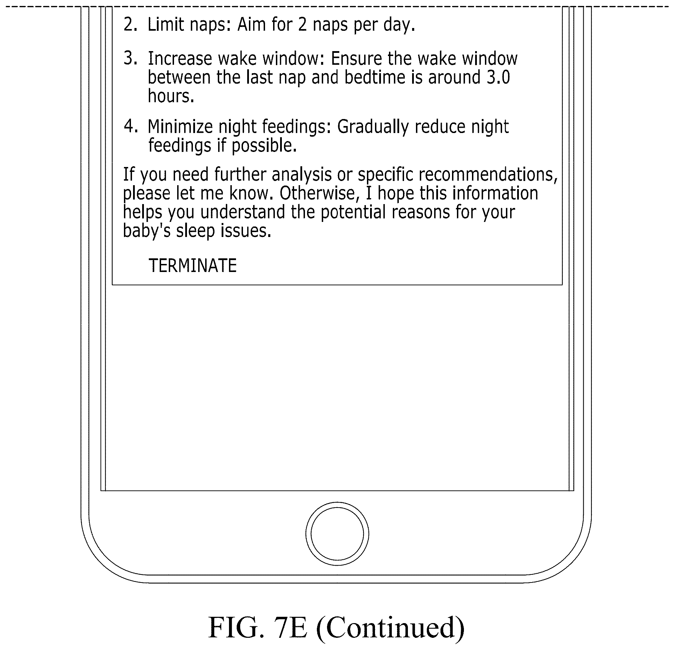

FIG. 7E illustrates an example content for responding to the query of FIG. 7A, as dynamically generated based on the executable code as described for FIG. 7B and the data file and descriptions as described for FIGs. 7C and 7D, and as transmitted to and displayed on the computing device of the user of FIG. 7A, in accordance with various embodiments disclosed herein.

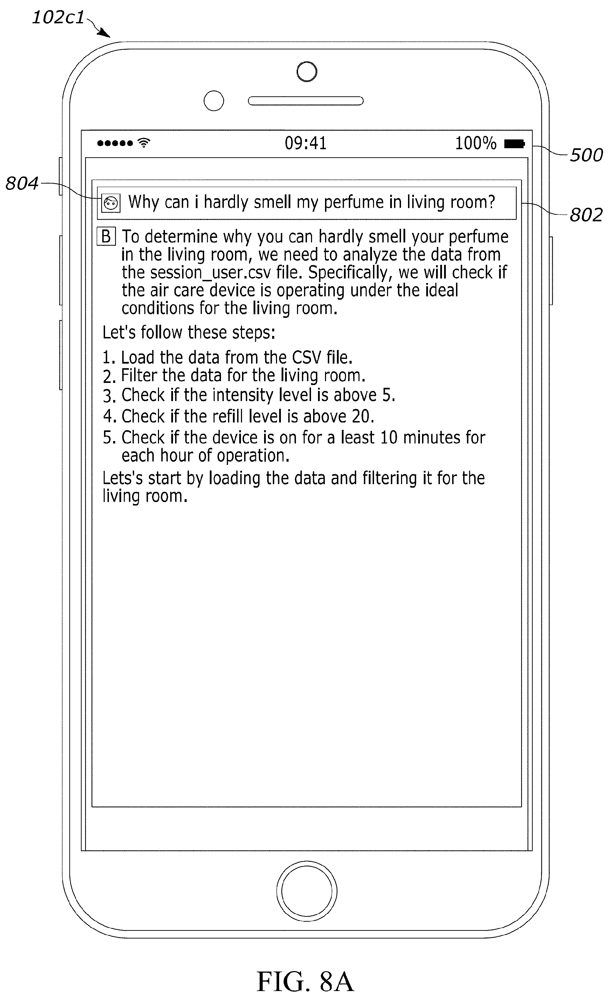

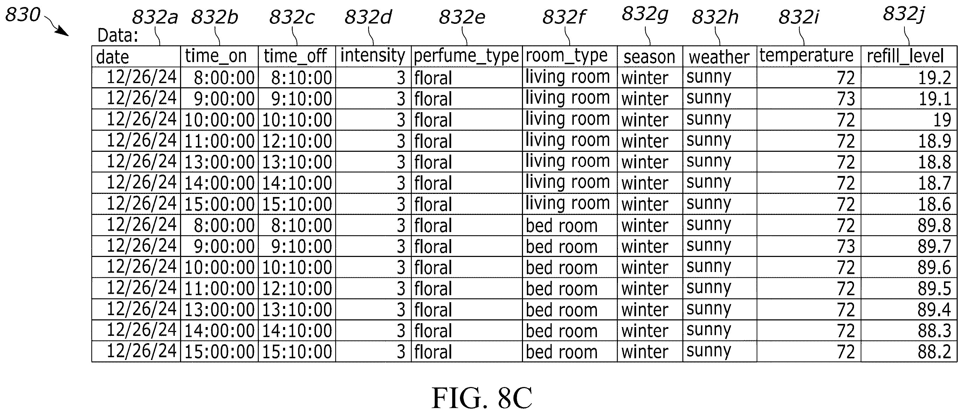

FIG. 8A illustrates an example user interface as rendered on a display screen of a computing device of a user and depicting an example query comprising natural language regarding a request regarding an air care use case (*e.g.*, as identifiable by a user's user-specific air care file as shown for FIG. 8C or otherwise herein), and in accordance with various embodiments disclosed herein.

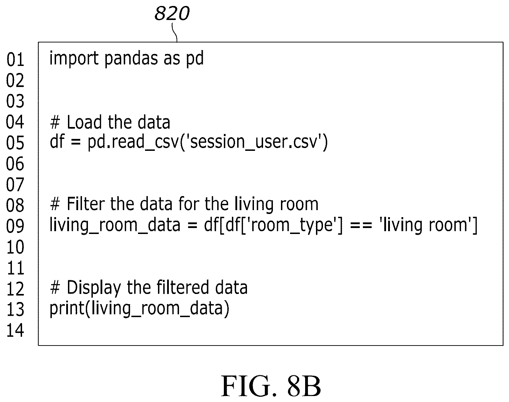

FIG. 8B illustrates an example of executable code comprising computing instructions as dynamically determined by an AI agent using the natural language of the query of FIG. 8A as input and programmed to address the air care use case as identified for FIG. 8A, in accordance with various embodiments disclosed herein.
FIG. 8C illustrates an example of a user-specific air care file as accessible by executable code as described by FIG. 8B, in accordance with various embodiments disclosed herein.

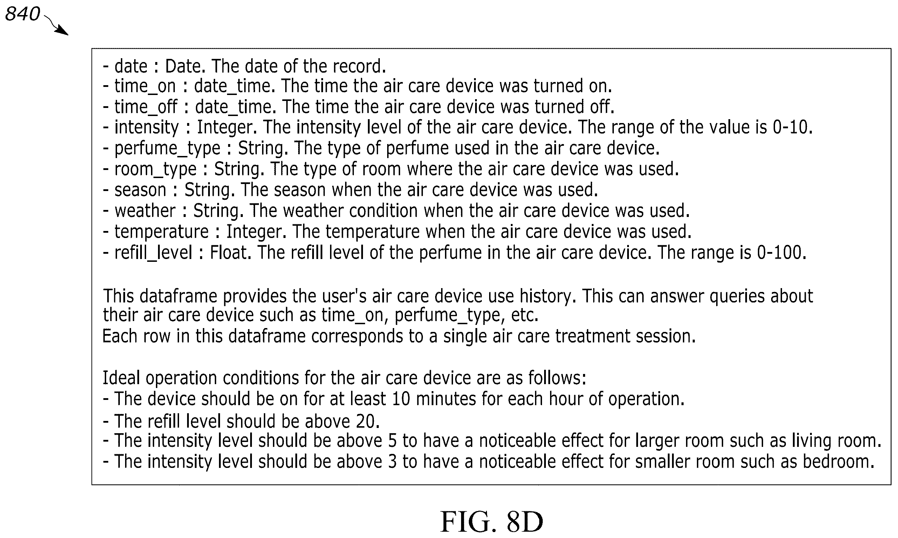

FIG. 8D illustrates an example of a pre-engineered prompt, or portion thereof, that defines descriptions of headers or metadata, including each of the headers or metadata of the user-specific air care file as described for FIG. 8C, where the pre-engineered prompt (or portion thereof) of FIG. 8C can be provided to an AI agent program that can provide the pre-engineered prompt (or portion thereof) to a generative AI model as described herein, in accordance with various embodiments disclosed herein.

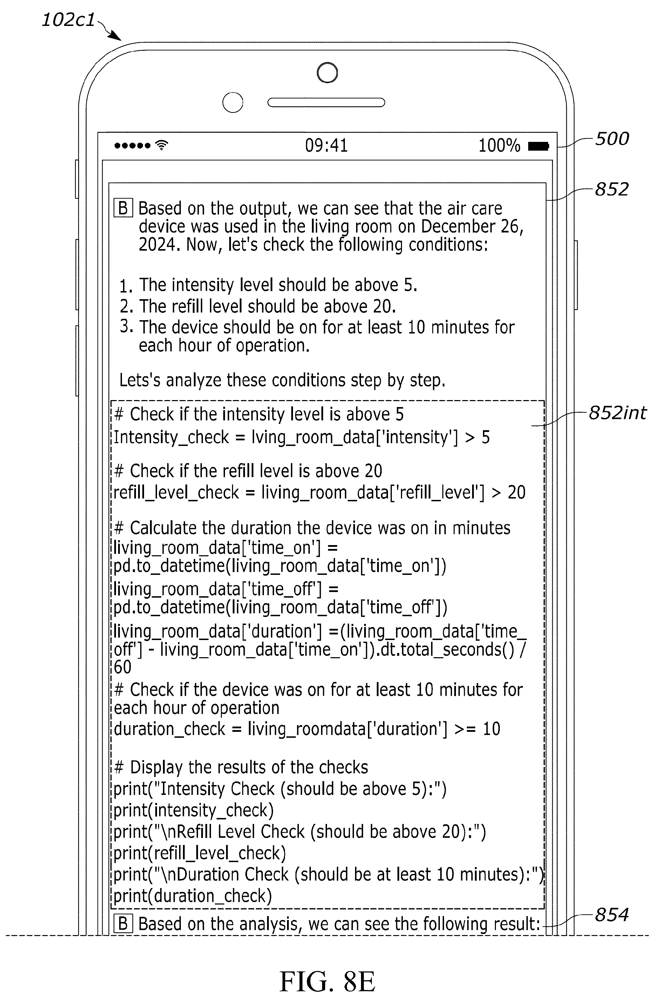

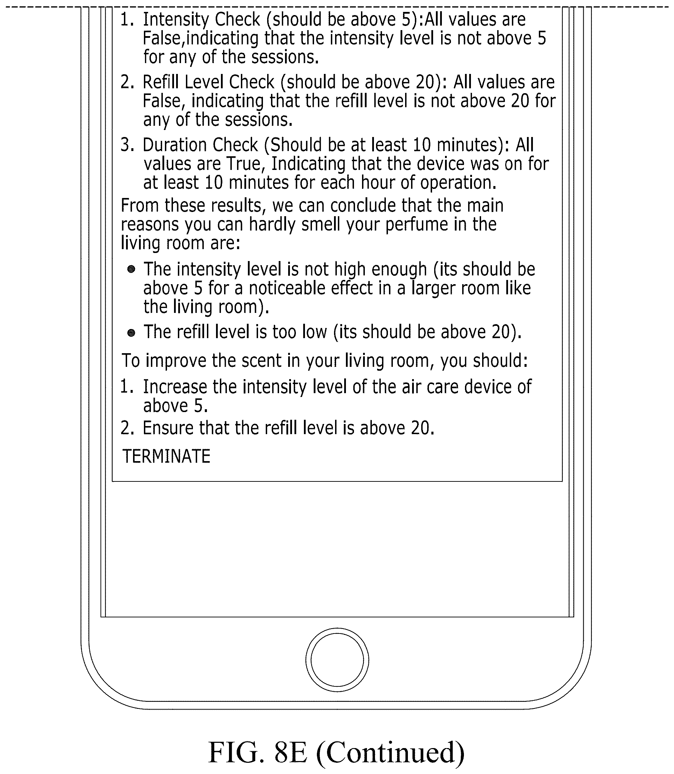

FIG. 8E illustrates an example content for responding to the query of FIG. 8A, as dynamically generated based on the executable code as described for FIG. 8B and the data file and descriptions as described for FIGs. 8C and 8D, including an intermediate executable code, and as transmitted to and displayed on the computing device of the user of FIG. 8A, in accordance with various embodiments disclosed herein.

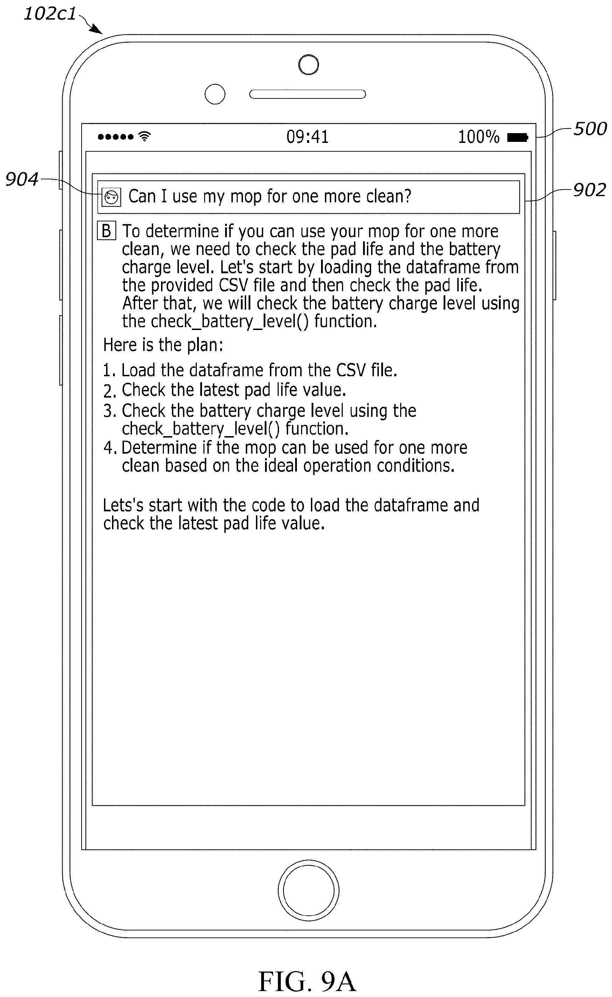

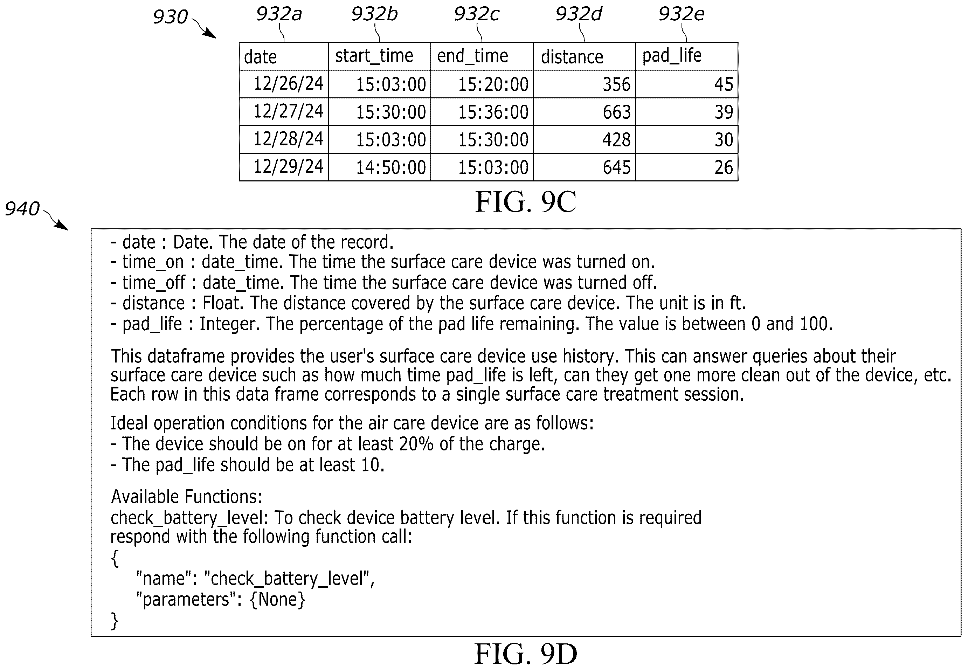

FIG. 9A illustrates an example user interface as rendered on a display screen of a computing device of a user and depicting an example query comprising natural language regarding a request regarding a surface care use case (*e.g.*, as identifiable by a user's user-specific surface care file as shown for FIG. 9C or otherwise herein), and in accordance with various embodiments disclosed herein.

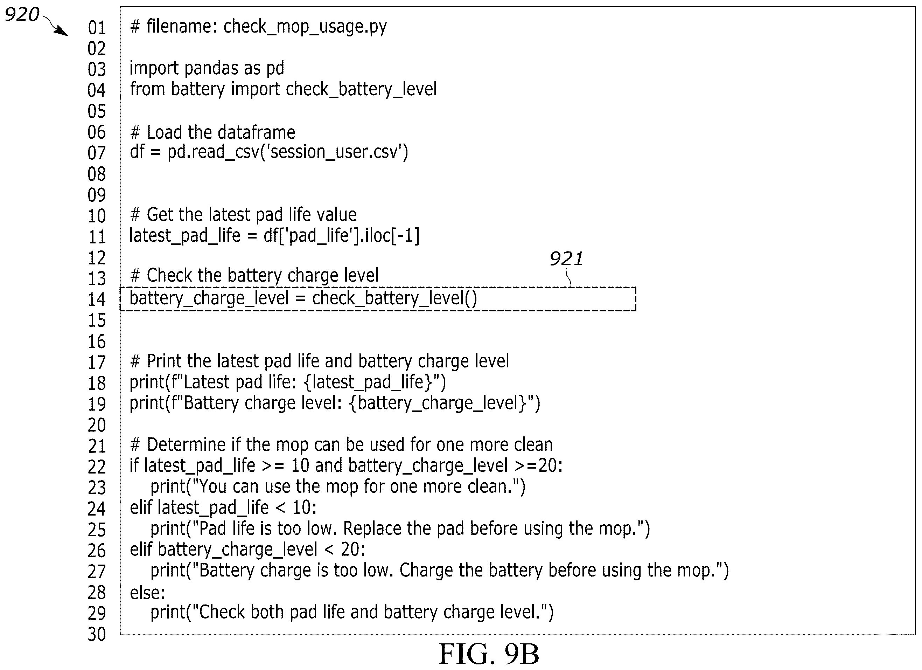

FIG. 9B illustrates an example of executable code comprising computing instructions as dynamically determined by an AI agent using the natural language of the query of FIG. 9A as input and programmed to address the surface care use case as identified for FIG. 9A, in accordance with various embodiments disclosed herein.
FIG. 9C illustrates an example of a user-specific surface care file as accessible by executable code as described by FIG. 9B, in accordance with various embodiments disclosed herein.
FIG. 9D illustrates an example of a pre-engineered prompt, or portion thereof, that defines descriptions of headers or metadata, including each of the headers or metadata of the user-specific surface care file as described for FIG. 9C, where the pre-engineered prompt (or portion thereof) of FIG. 9C can be provided to an AI agent program that can provide the pre-engineered prompt (or portion thereof) to a generative AI model as described herein, in accordance with various embodiments disclosed herein.

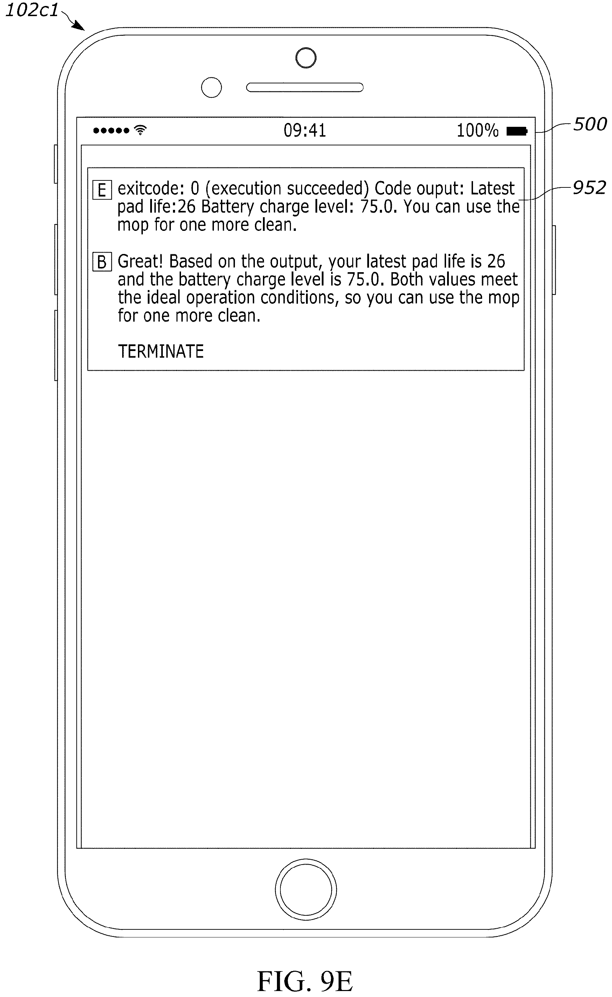

FIG. 9E illustrates an example content for responding to the query of FIG. 9A, as dynamically generated based on the executable code as described for FIG. 9B and the data file and descriptions as described for FIGs. 9C and 9D, and as transmitted to and displayed on the computing device of the user of FIG. 9A, in accordance with various embodiments disclosed herein.

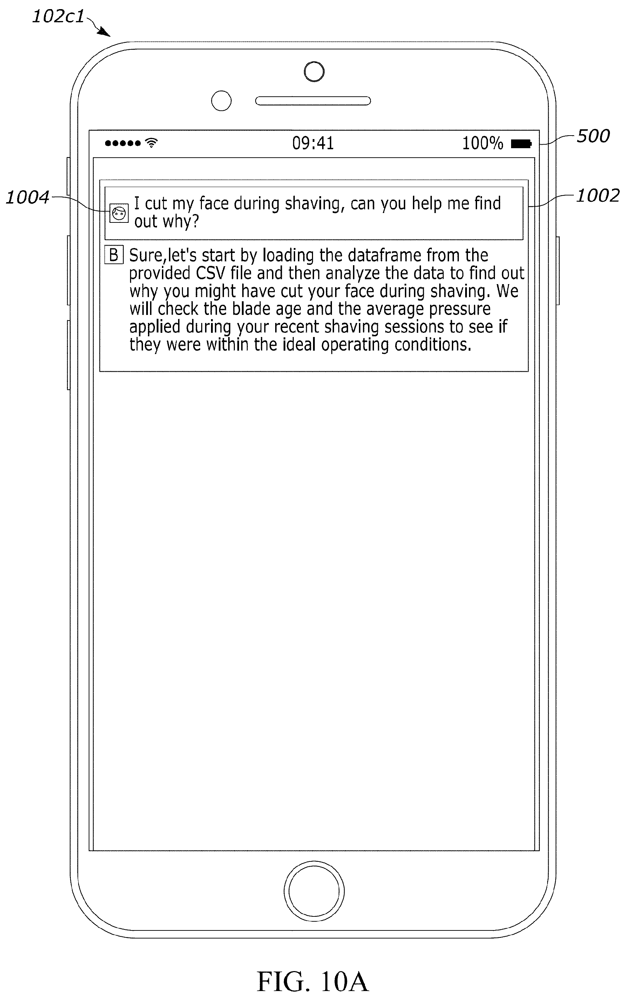

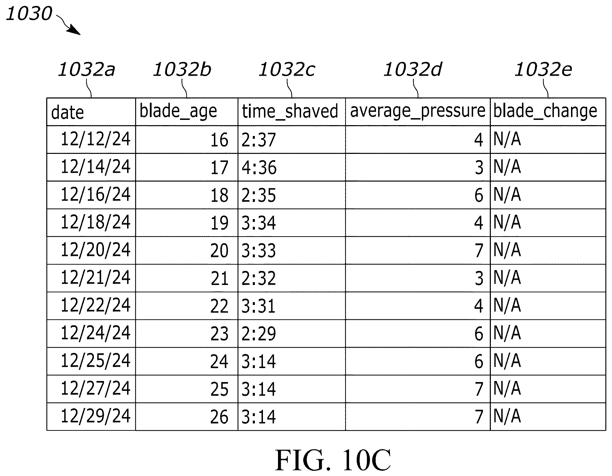

FIG. 10A illustrates an example user interface as rendered on a display screen of a computing device of a user and depicting an example query comprising natural language regarding a request regarding a grooming care use case (*e.g.*, as identifiable by a user's user-specific grooming care file as shown for FIG. 10C or otherwise herein), and in accordance with various embodiments disclosed herein.

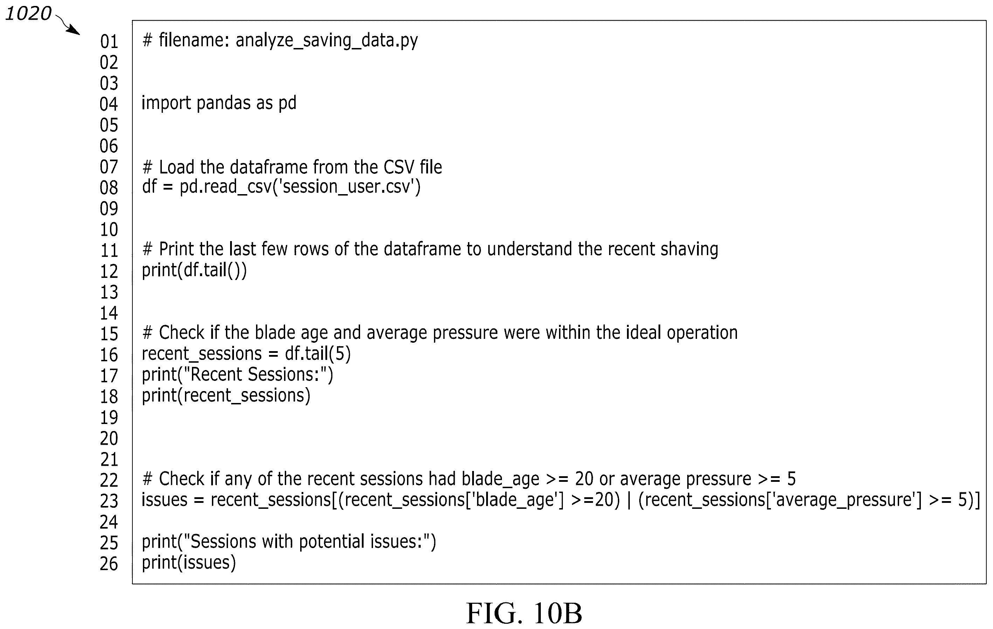

FIG. 10B illustrates an example of executable code comprising computing instructions as dynamically determined by an AI agent using the natural language of the query of FIG. 10A as input and programmed to address the grooming care use case as identified for FIG. 10A, in accordance with various embodiments disclosed herein.
FIG. 10C illustrates an example of a user-specific grooming care file as accessible by executable code as described by FIG. 10B, in accordance with various embodiments disclosed herein.

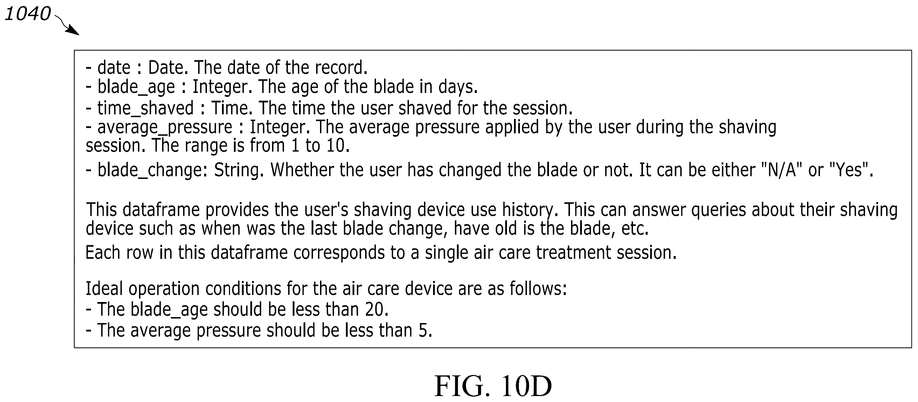

FIG. 10D illustrates an example of a pre-engineered prompt, or portion thereof, that defines descriptions of headers or metadata, including each of the headers or metadata of the user-specific grooming care file as described for FIG. 10C, where the pre-engineered prompt (or portion thereof) of FIG. 10C can be provided to an AI agent program that can provide the pre-engineered prompt (or portion thereof) to a generative AI model as described herein, in accordance with various embodiments disclosed herein.

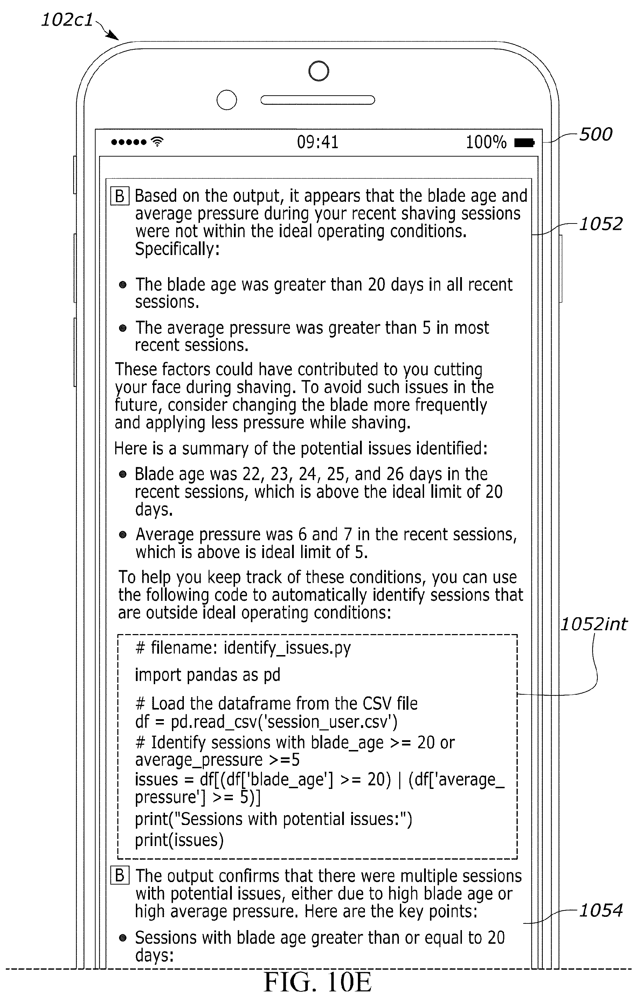

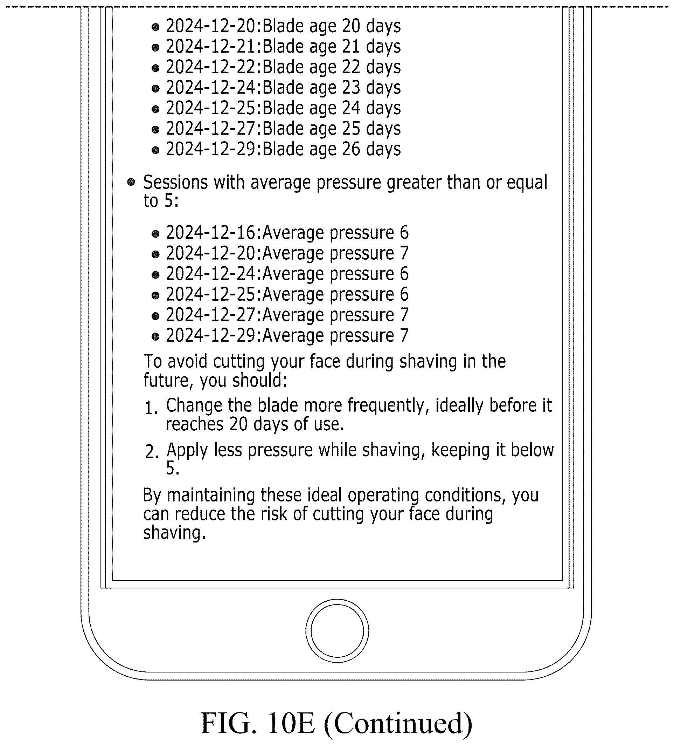

FIG. 10E illustrates an example content for responding to the query of FIG. 10A, as dynamically generated based on the executable code as described for FIG. 10B and the data file and descriptions as described for FIGs. 10C and 10D, including an intermediate executable code, and as transmitted to and displayed on the computing device of the user of FIG. 10A, in accordance with various embodiments disclosed herein.

The Figures depict preferred embodiments for purposes of illustration only. Alternative embodiments of the systems and methods illustrated herein may be employed without departing from the principles of the invention described herein.

### DETAILED DESCRIPTION OF THE INVENTION

FIG. 1 illustrates an example artificial intelligence (AI) agent system 100 configured to dynamically generate content regarding oral activity, in accordance with various embodiments disclosed herein. As shown in the example of FIG. 1, server 110 is configured to receive queries from users (*e.g.*, user 101). The query may be received from an oral care implement 102 (*e.g.*, an electronic toothbrush), a docking station of the oral care implement, and/or otherwise an input device. An input device may comprise, for example, a voice enabled assistant device for receiving voice data, a camera for receiving images or video, and/or a keyboard for receiving text data. The keyboard may comprise a physical keyboard or a virtual keyboard as displayed on a user interface of a computing device (*e.g*., as shown for Figures 5A, 6A, or elsewhere herein). In various aspects, input may comprise voice, images or video, and/or text, and may be received at server 110. Further, in various aspects the input, *e.g*., input of the user 101, may comprise natural language including natural language tokens defining oral activity (*e.g.*, tooth brushing) of the user (*e.g.*, user 101). For example, the input may comprise voice input, which can be analyzed to generate natural language tokens.

With further reference to FIG. 1, server 110 of AI agent system 100 may comprise one or more computer servers. In various embodiments server 110 may comprise multiple servers, which may comprise multiple, redundant, or replicated servers as part of a server farm. In still further embodiments, server 110 may be implemented as cloud-based servers, such as a cloud-based computing platform. For example, server 110 may be any one or more cloud-based platform(s) such as MICROSOFT AZURE, AMAZON AWS, or the like.

Server 110 may include one or more processors (*i.e.*, CPU(s)) as well as one or more computer memories. The memories of the server may comprise one or more forms of volatile and/or non-volatile, fixed and/or removable memory, such as read-only memory (ROM), electronic programmable read-only memory (EPROM), random access memory (RAM), erasable electronic programmable read-only memory (EEPROM), and/or other hard drives, flash memory, MicroSD cards, and others. memory may store an operating system (OS) (*e.g*., Microsoft Windows, Linux, UNIX, etc.) capable of facilitating the functionalities, apps, methods, or other software as discussed herein. The computer memory of server 110 may also comprise a database, or one or more databases (*e.g.*, a relational database, such as Oracle, DB2, MySQL, or a NoSQL based database, such as MongoDB). The one or more processors of server 110 may interface with the memory and/or database(s) of server 110 via a computer bus to create, read, update, delete, or otherwise access or interact with the data stored in memories and/or the database. The data stored in the memories and/or database(s) may include all or part of any of the data or information described herein, including, for example, data files, code, or other information or data as otherwise described herein.

The memories of server 110 may store machine readable instructions, including any of one or more application(s) or programs (*e.g*., one or more AI agent programs as described herein), one or more oral activity files (*e.g.,* such as user-specific tooth brushing behavior files), predefined queries, applications, executable code, and/or other computer code which may be implemented to facilitate or perform the features, functions, or other disclosure described herein, such as any methods, processes, elements or limitations, as illustrated, depicted, or described for the various flowcharts, illustrations, diagrams, figures, and/or other disclosure herein. Such machine-readable instructions are executable by the one or more processors of server 110. For example, the one or more processors of server 110 may be connected to the memories via a computer bus responsible for transmitting electronic data, data packets, or otherwise electronic signals to and from the processor and memories in order to implement or perform the machine-readable instructions, methods, processes, elements or limitations, as illustrated, depicted, or described for the various flowcharts, illustrations, diagrams, figures, and/or other disclosure herein.

Server 110 may further include a communication component configured to communicate (*e.g.*, send and receive) data via one or more external/network port(s) to one or more networks or local terminals, such as a computer network and/or a terminal for sending and receiving data, such as a query and/or content described herein. In some embodiments, server 110 may include a client-server platform technology such as ASP.NET, Java J2EE, Ruby on Rails, Node.js, a web service or online application programming interface (API), responsive for receiving and responding to electronic requests. The server 110 may implement the client-server platform technology that may interact, via the computer bus, with the memories(s) (including the applications(s), component(s), API(s), data, etc. stored therein) and/or database to implement or perform the machine readable instructions, methods, processes, elements or limitations, as illustrated, depicted, or described for the various flowcharts, illustrations, diagrams, figures, and/or other disclosure herein.

In various embodiments, server 110 may include, or interact with, one or more transceivers (*e.g*., WWAN, WLAN, and/or WPAN transceivers) functioning in accordance with IEEE standards, 3GPP standards, or other standards, and that may be used in receipt and transmission of data via external/network ports connected to a computer network. In some embodiments, computer network may comprise a private network or local area network (LAN). Additionally, or alternatively, a computer network may comprise a public network such as the Internet.

As described herein, in some embodiments, server 110 may perform the functionalities as discussed herein as part of a cloud network or may otherwise communicate with other hardware or software components within the cloud to send, retrieve, or otherwise analyze data or information described herein.

In general, a computer program, such as an AI agent program, or code (*e.g*., executable code programmed to address oral activity as described herein) may be stored on a computer usable storage medium, or tangible, non-transitory computer-readable medium (*e.g*., standard random access memory (RAM), an optical disc, a universal serial bus (USB) drive, or the like) having such computer-readable program code or computer instructions embodied therein, wherein the computer-readable program code or computer instructions may be installed on or otherwise adapted to be executed by one or more processors processor (*e.g*., working in connection with the respective operating system in memories) to facilitate, implement, or perform the machine readable instructions, methods, processes, elements or limitations, as illustrated, depicted, or described for the various flowcharts, illustrations, diagrams, figures, and/or other disclosure herein. In this regard, the program code may be implemented in any desired program language, and may be implemented as machine code, assembly code, byte code, interpretable source code or the like (*e.g.*, via Golang, Python, C, C++, C#, Objective-C, Java, Scala, ActionScript, JavaScript, HTML, CSS, XML, etc.).

In various aspects, server 110 may receive a query (*e.g.*, query 101q) of a user (*e.g.*, user 101) comprising natural language including natural language tokens defining oral activity. In the example of FIG. 1, the query 101q from the user is a question by the user: "Did I miss any zone?" referring to zones of the user's mouth. An AI agent (*e.g.*, AI agent program 122) may be executed to address the query (*e.g*., query 101q). In some aspects, the AI agent is selected from a plurality of AI agents by an AI Agent router (*e.g*., AI agent router 120 as described for FIG. 2). The AI agent router 120 may have selected the AI agent program 122 because of the ability of AI agent program 122 to address the query (*e.g*., query 101q) as determined based on analysis of the natural language and its respective natural language tokens. For example, AI agent program 122 may be configured to address zones of mouths and may be selected by AI Agent router to generate content, which may include a text-based response to query 101q. In the example of FIG. 1, content 130 comprises text-based content (*e.g*., "Based on the information provided, the zones that you've missing are zones 1 and 3") as dynamically generated by AI agent program 122. Such dynamically generated content can be generated and returned to server 110 (*e.g.,* via an AI agent program 122 directly or through AI agent router 120) for transmission to a computing device of the user for output by the computing device regarding the oral activity of the user. For example, transmission may include transmission 114t, generation, and return 114r via an AI agent router (e.g., AI Agent program 122), for example, as described herein. Additionally, or alternatively, transmission may include direct transmission and return 114d to an AI agent (e.g., AI agent 122) without the use of an AI agent router (e.g., without any interaction with AI agent router 120).

In some aspects, the user may send, from his or her computing device, additional data to server 110. Such data may comprise brushing zone data (e.g., included in brush behavior data 106), which may be included as part of a file (*e.g.,* an oral activity file and/or user-specific tooth brushing behavior file as described herein for FIG. 4). Such data may be stored on the memorie(s) and/or database of server 110.

In some aspects, a computing device (*e.g*., computing device 102c) may comprise an oral care implement, a docking station of an oral care implement, and/or another computing device, such as a phone. For example, a computing device may comprise mobile devices and/or client devices for accessing and/or communications with server 110. Such computing devices may comprise one or more processor(s) and/or an imaging device for capturing images or video. In various embodiments, user computing devices may comprise a mobile phone (*e.g*., a cellular phone), a tablet device, a personal data assistance (PDA), or the like, including, by non-limiting example, an APPLE IPHONE, IPAD device, or a GOOGLE ANDROID based mobile phone or table.

Computing devices may comprise a wireless transceiver to receive and transmit wireless communications and/or to and from base stations. In various embodiments, queries, content, and/or other data as described herein may be transmitted via computer network to server 110 for implementation and/or execution algorithms and/or methods as described herein.

Still further, computing devices may include a display screen for displaying graphics, images, text, product(s), data, pixels, features, and/or other such visualizations or information as described herein. In various embodiments, graphics, images, text, product(s), data, pixels, features, and/or other such visualizations or information may be received from server 110 for display on the display screen of the computer devices for example, as described for FIGs. 5A, 5C, 6A, 6C, and/or 6D. Additionally, or alternatively, a user computer device, *e.g*., as described herein for FIGs. 5A, 5C, 6A, 6C, and/or 6D, may comprise, implement, have access to, render, or otherwise expose, at least in part, an interface or a guided user interface (GUI) for displaying text and/or images on its display screen.

FIG. 2 illustrates an example an AI agent router 120 configured to select an AI agent program (*e.g.* AI agent program 122) from one or more AI agent programs (*e.g.,* AI agent programs 121, 122, 123, or 124) based on natural language tokens of a query (*e.g*., the query 101q as described for FIG. 1 or elsewhere herein). The AI agent router 120 is configured to route the query to the AI agent program (*e.g*. AI agent program 122) for providing output for the query, in accordance with various embodiments disclosed herein.

In various aspects, AI agent router 120 may comprise computing instructions executable on one or more processors, such as the one or more processors described herein for FIG. 1. This may include one or more processors of sever 110 and/or computing device 102c . Additionally, or alternatively, AI agent router 120 may comprise a hardware router or device, with computing instructions stored on a memory thereon, and communicatively coupled (*e.g*., via a network) to a computing device (*e.g.*, computing device 102c) or server (*e.g.*, server 110) for receiving, routing, replying, and/or otherwise transmitting queries, natural language tokens, content, and/or other data or information as described herein.

It is to be understood that implementation of AI agent router 120 is optional. For example, in some implementations, an AI agent program (*e.g*., AI agent program 122) can be invoked directly, without using an AI agent router (*e.g*., an AI agent router 120).

In the example of FIG. 2, AI agent router 120 is utilized or otherwise implemented. In various aspects, AI agent router 120 can be implemented as part of algorithm or method, including, for example, as part of AI agent method 300 as described for FIG. 3 herein.

With reference to FIG. 2, a query comprising natural language and/or natural language tokens, as detected for the query (*e.g*., query 101q), is provided or provided to the AI agent router 120. As shown in the example of FIG. 2, the query is provided from a user (*e.g*., user 101 as described for FIG. 1). The method (*e.g*., as part of AI agent method 300) comprises analyzing, by an AI agent router (*e.g.*, AI agent router 120) executing on the one or more processors (*e.g.*, processor(s) of server 110), the natural language tokens to select the AI agent program (*e.g*., AI agent program 122) from one or more AI agent programs (*e.g.*, any one of AI agent programs 121-124). Each of the AI agent programs are configured to provide output for specific types of queries. The AI agent router may then route the query to the AI agent program as selected to handle the type of query indicated by the natural language tokens. The AI agent router may also receive output (*e.g*., content) from the agent (e.g., as dynamically generated by a generative AI model) for transmitting such output back to a computing device of the user (*e.g*., user 101) as described herein.

In the example of FIG. 2, the query of user 101 is transmitted to AI agent router 120. AI agent router 120 then analyzes the natural language tokens of the query to select AI agent program 122. The query may have been related to a bruising question (e.g.: "Did I miss any zones" as described for FIG. 1). The query may be broken down into natural language tokens, and based on the tokens AI agent router 120 may select AI agent program 122, which comprises a brushing agent coder (e.g., coder 120coder) for receiving the query. Coder 122coder may comprise program code, for example, either compiled code or code for interpretation by the one or more processors as described herein. The AI agent program 122 may be selected because it is known to address the specific type of query and/or user-specific tooth brushing behavior as identified in the natural language tokens. Brushing agent coder 122coder of AI agent program 122 may dynamically determine executable code for addressing oral activity as determined from the natural language tokens. The AI agent program 122 may then invoke a code executor 122ce to execute the executable code to address the oral activity. In the example of FIG. 2, this may comprise accessing the user's brush history 122bh, which may be stored in computer memory as an oral activity file (*e.g.*, a user-specific tooth brushing behavior file 400 of the user 101, for example, as described for FIG. 4 herein). Execution of the executable code causes the AI agent program 122 and/or the one or mor processors executing computing instructions to dynamically generate content for transmission back to a computing device of the user regarding the oral activity, for example, as further described herein for FIG. 3.

FIG. 3 illustrates an example artificial intelligence (AI) agent method 300 for dynamically generating content regarding oral activity, in accordance with various embodiments disclosed herein. At block 310, method 300 comprises receiving, by one or more processors (*e.g*., of server 110) , a query comprising natural language including natural language tokens defining oral activity of a user. Natural language tokens may be generated by implementation of a tokenization algorithm on the text or otherwise data of the query. In some aspects herein, tokenization refers to implementation of a Natural Language Processing (NLP) algorithm, which comprises the process of converting a sequence of text into smaller parts, known as tokens. These tokens can be as small as characters or syllables of words or as long as words themselves. In such aspects, tokenization, and the tokens as output as a result, allows a computing device to understand human language by breaking down words into smaller portions (*e.g*., syllables or characters), which are less compute intensive for the computing device to analyze and process. Tokens may be used by artificial intelligence models (*e.g*., machine learning models), including generative artificial intelligence models, to determine and analyze text, such as queries of users or oral care implements.

Additionally, or alternatively, as used herein the terms "token" (or "natural language token") may have an alternative meaning. More generally, a token (or natural language token) may be or otherwise comprise a unit derivable from natural language. In some aspects, a unit may comprise syllables or characters or words; in other aspects, the unit may comprise computer bytes or patches of computer bytes, referred to herein as patches. For example, bytes may be encoded into dynamically sized patches, which serve as the primary units of computation for training generative AI model as described herein. For example, in one example aspect, a Byte Latent Transformer (BLT) can encode bytes into dynamically sized patches, as described by the example article Pagnoni et al., Byte Latent Transformer: Patches Scale Better Than Tokens (Dec. 12, 2024), which is incorporated in its entirety by reference herein. Herein, with respect to embodiments that use a BLT, the term token (or natural language token) is used to refer to a patch as encoded or otherwise created by the BLT. Herein, with respect to embodiments that use a BLT, the term "tokenization" is used to refer to as encoding bytes to create a given patch by the BLT. It is to be understood that the use of the BLT and its tokenization process by creating patches of data is one example of tokenization for use in training an AI model, such as a generative AI model as described herein. Accordingly, in some aspects, natural language tokens comprise units of computation. Additionally, or alternatively, in some aspects natural language tokens may comprise bytes or patches.

More generally, tokenization is the process of converting raw input data-such as text, audio, images, or video-into a structured format that can be effectively processed by machine learning models. This involves breaking down the input into smaller, manageable units known as tokens or patches. The method of tokenization can vary significantly based on the approach used. For natural language, tokens can be as small as characters or syllables of words or as long as words themselves. For bytes, tokens can be units of computation or otherwise patches.

In some aspects, a query may comprise a user query of a user (*e.g*., user 101). Such a query may be provided as text input into a prompt on a user interface of a computing device (*e.g*., computing device 102c1 as described for FIGs. 5A, 5C, 6A, 6C, and/or 6D herein).

In other aspects, the query may comprise a system query invoked by one or more processors (*e.g.*, one or more processors of server 110 and/ or of computing device 102c1). In such aspects, the system query may comprise a predefined query stored in a computer memory, *e.g*., a computer memory of server 110 and/or of a computing device (*e.g*., computing device 102c1). The system query and/or predefined query may comprise a commonly asked or otherwise invoked query, such as "analyze my brushing technique" or the like. In some aspects, the system query (*e.g*., the predefined query) may be linked to a graphic selectable by the user from a graphic user interface (GUI) displayed on a display screen. For example, the graphic may comprise a button selectable on the GUI. When the graphic (*e.g*., button) is selected, the one or more processors invoke the system query, *e.g*., by sending it to an app on computing device 102c and/or server 110 for analysis and/or execution by an AI agent program (*e.g*., AI agent program 122), for example, as described herein.

In some aspects, the natural language of the query may be provided by, or otherwise comprise, text input or audible voice input where the natural language tokens comprise text-based tokens or audio tokens. For example, the query can be a text query or an audible voice query. The query may be, for example, "Can you show me where I under brush in a picture?" (*see e.g.,* Figure 5A), where the text of the query is analyzed, and natural language tokens are generated therefrom.

In some aspects, receiving audio input and converting such audio input into natural language tokens comprises receiving audio input (*e.g*., voice input) and providing such input into an LLM model trained to covert audio input to text output, and generating tokens from the text of the converted audio input. In another aspect, receiving audio input and converting such audio input into natural language tokens comprises end-to-end speech input and streaming audio output. In such aspects, the audio or otherwise speech may be input into a multi-modal model trained on audio token to speech and/or audio output. In such aspects, the audio is converted directly to natural language tokens without first converting the audio into text.

In various aspects, a text or audio input may be received by various devices, *e.g*., such as computing device 102c as shown in FIG. 1. For example, a text-based query or audio-based query may be received from an oral health care application (app), such as an oral health chatbot app, installed on and/or executing on a hub-based device and/or a mobile device (*e.g*., mobile phone), the latter as shown and described for FIGs. 5A, 5C, 6A, 6C, and/or 6D. Additionally, or alternatively, receipt and analysis of a text-based query may comprise an oral care companion device with touch screen, *e.g.,* where pressing button triggers a pre-set inquiry, *e.g.,* system query as described herein). Still further, additionally or alternatively, receipt and analysis of an audio-based query may comprise an audio inquiry to a smart toothbrush base station (*e.g*., as illustrated for example by computing device 102c) featuring a microphone and a speaker for receiving the voice input.

In some aspects, the query may comprise receiving a video depicting brushing activity of a user during a brushing session. The query may refer to one or more images, which may comprise multimedia tokens (*e.g*., pixel data as determined from images). In such aspects, the query may include natural language about the video, *e.g.,* a query requesting a brushing analysis of the user based on the pixel data of images captured of the user while the user brushes his or her teeth with an oral care implement (*e.g.*, an electronic toothbrush). With respect to such aspects, a user (*e.g.*, user 101) may upload a video and then ask or otherwise submit a query about the video, *e.g*., which may comprise a query about a video showing the user's teeth brushing style or technique. Such query may comprise, by way of non-limiting example, the following: "analyze whether my brushing pattern is correct." The AI agent program (*e.g*., AI agent program 122) may then respond to the user by dynamically generating content, *e.g*., text, stating that the video shows the user using a side-to-side brushing technique (as determined from the pixel data from the images of the video). The content could also recommend that the user improve their brushing technique by adopting an up-and-down brushing technique.

In some aspects, the system query (*e.g*., predefined query) may be invoked periodically. In such aspects, for example, the one or more processors may periodically trigger the AI agent program to analyze or otherwise review the user brushing behavior and provide summaries and/or guidance to the user to help influence, guide, or otherwise change user behavior, such as improvement in brushing technique or otherwise use or purchase of an oral care instrument for improving user brushing results.

At block 320, method 300 comprises dynamically determining, by an AI agent program (*e.g.*, AI agent program 122) executing on the one or more processors and using the natural language of the query as input, executable code programmed to address the oral activity. The executable code may comprise compiled code or interpretable code, which may comprise computer code as programmed in one or more computing languages such as one or more of Python, C++, C#, Java, and/or the Go program language(s). Still further, the AI agent program (e.g., AI agent program 122) comprises computing instructions stored on a computer memory , and wherein one or more oral activity files are stored on a computer memory (*e.g*., a memory of server 110 and/or computing device 102c1). Each oral activity file may comprise data related to oral activity of respective users during respective oral activity sessions. For example, an oral activity file may comprise a user-specific tooth brushing behavior file 400 as described for FIG. 4 herein.

In various aspects, dynamically determining the executable code comprises dynamically identifying preexisting executable code stored on the computer memory and programmed to address a specific query. The identification of the preexisting executable code may comprise identifying program code to address a common question. In some aspect, the preexisting executable code may be linked to a system query (*e.g*., a predefined query as described herein).

In other aspects, dynamically determining the executable code comprises dynamically generating new executable code created to address the oral activity. In such aspects, the new executable code may comprise code does not exist that is newly generated to address a new and/or different query, or at least a query that is not linked to a preexisting executable code.

In some aspects, method 300 may comprise analyzing video input. Such aspects may comprise receiving a video depicting brushing activity during a brushing session and comprising multimedia tokens (*e.g*., pixel data within images of the video). In such aspects, a user's query comprising the natural language may include a query about the video. For example, the query may include natural language about the video, *e.g.,* a query requesting a brushing analysis of the user based on the pixel data of images captured of the user while the user brushes his or her teeth with an oral care implement. In such aspects, method 300 may comprise analyzing, by execution of the executable code, the multimedia tokens to address the oral activity. The content as output for responding to query (as described herein) can be based at least in part on the brushing activity depicted in the video. In some aspects, the video may comprise image frames of the user, e.g., engaged in an oral activity such as brushing teeth). In some aspects, the video may be captured by a second user (*e.g.*, a parent capturing video of the user of the oral are implement, *e.g.,* a child). In such aspects, the video can be captured by a second user. For example, in such an aspect, one user (*e.g.*, a parent) takes the video of a user (e.g., a child) brushing and the query is about the child's brushing technique and/or brushing history.

At block 330, method 300 comprises dynamically generating, based on execution of the executable code by the one or more processors, content as output for responding to the query. In various aspects, the content as dynamically generated based on the execution of the executable code may comprise one of a chart, a graphic, a textual output, audible output, and/or a video. In various aspects, an AI agent program (*e.g*., AI agent program 122) executing the executable code is configured to provide output for the query. The AI agent program itself and/or an AI agent router (*e.g.*, AI Agent program 122) may then route or transmit the output (*e.g.*, content) back to a computing device (*e.g.*, computing device 102c and/or 102c1) of the user (*e.g.*, user 101), where such output may comprise content and/or modified settings 106o, or other output as described herein.

In some aspects, prior to dynamically generating the content as output, one or more intermediate data, executable code, or graphics may be generated. The content may be generated based, at least in part, on the intermediate data, executable code, or graphics. In one example, dynamically generated content may comprise a video, where, for example, the video could comprise a video of a dentist or avatar (*see e.g.*, avatar 642 of Fig. 6D herein) providing brushing advice or guidance specific to the user. In this example, an avatar (*e.g*., avatar 642) of a dentist could be generated as an immediate graphic for use in video content. Such video content may comprise controlled lip synchronization, expression(s), and/or hand motion(s) as the video of the avatar of the dentist as the dentist provides guidance in audio and video output to the user via a display of a computing device (*e.g*., computing device 102c).

In various aspects, an AI agent program (*e.g*., AI agent program 122) comprises, or is configured to access, a generative AI model for dynamically generating or determining at least one of the executable code or the content. The generative AI model may comprise a cloud-based generative AI model such as the GPT series generative AI model by OPENAI or the CLAUDE GenAI generative AI model by ANTHROPIC, each accessible via a computer network by a server (e.g., server 110) and/or computing device 102c/102c1. In other aspects, the generative AI model may comprise a local generative AI model such as the LLAMA generative AI model by META or the Mistral generative AI model, each of which may be downloaded and installed and accessed on server 110 and/or computing device 102c/102c1. It is to be understood that any multimodal AI models and/or language models can also be used including, but not limited to, large reasoning models and image generation models such as STABLE DIFFUSION or video generation model such as SORA or the like.

More generally, generative AI comprises a type of AI designed to generate new content such as text, images, audio, or code by leveraging patterns and structures learned from vast datasets. A generative AI model can be trained via AI models such neural networks and/or a large language model (LLM), which are trained through a process called supervised or unsupervised learning. During training, the generative AI model analyzes large amounts of data to identify relationships and generate outputs that mimic the style and structure of the input data. Once trained, generative AI model, such as the generative AI model described herein, can use prompts-specific instructions or examples provided by users-as input to guide its content generation, including the output (*e.g*., text, videos, and/or audio described herein). By analyzing the prompt, the AI activates its learned patterns to produce relevant and coherent outputs (*e.g*., content), ranging from written explanations to creative visual designs, tailored to the given context. In various aspects herein, the prompts may be generated by AI agent programs and provided as input into a generative AI model.

In one example, the generative AI model may be configured to receive an input prompt and can generate content based on the prompt. In some aspects, an AI agent program (*e.g*., AI agent program 122) provides as input a pre-engineered prompt to the generative AI model. In response, the generative AI model generates or determines at least one of the executable code or the content based on the pre-engineered prompt.

In another example, the AI agent program (e.g., AI agent program 122) may access a user-specific tooth brushing behavior file 400 (as shown for FIG. 4) in order to dynamically determine content. In such an example, method 300 may comprise accessing a user-specific tooth brushing behavior file 400 of the user (*e.g.*, user 101) that is stored on the computer memory (*e.g.*, a memory of server 110). The user-specific tooth brushing behavior file 400 may define user-specific tooth brushing metrics of the user (*e.g*., user 101) when the user brushed during respective user tooth brushing sessions. The user-specific tooth brushing metrics may define a plurality of mouth zones (*e.g.*, top left, top center, top right, bottom left, bottom center, and bottom right as shown for FIG. 5C herein). In such examples, the query can comprise the natural language tokens related to oral activity of the user, and the AI agent program can comprise a brushing AI agent program (*e.g*., AI agent program 122). In such aspects, computing instructions, executing on the one or more processors (*e.g*., of server 110 and/or computing device 102c1) implements the AI agent method 300 by accessing the user-specific tooth brushing metrics (*e.g*., in the user-specific tooth brushing behavior file 400). The computing instructions can also invoke the brushing AI agent program to dynamically determine the executable code to analyze the user-specific tooth brushing behavior file to determine and interpret data from the user-specific tooth brushing behavior file 400 . This may include any one or more one of: (a) brushing times of the user across the plurality of mouth zones; (b) brushing pressure at any one or more of the plurality of mouth zones; (c) brushing movement patterns at any one or more of the plurality of mouth zones; and/or (d) brushing dwell times at any one or more of the plurality of mouth zones, and/or any other user-specific tooth brushing behavior as described herein.

FIG. 4 illustrates an example user-specific tooth brushing behavior file 400 or otherwise the oral activity file of a user, in accordance with various embodiments disclosed herein. In various aspects, a user-specific tooth brushing behavior file 400 may comprise data comprising brushing sessions or other oral related data captured, for example, defining when a user changes a brush head. As shown for FIG. 4, the user-specific tooth brushing behavior file 400 comprises brushing behavior history of a user. An AI agent program (*e.g*., AI agent program 122) may access and use the user-specific tooth brushing behavior file 400 to generate personalized brushing content as described herein. As shown for FIG. 4, the user-specific tooth brushing behavior file 400 comprises headers or metadata identifying each of the plurality of mouth zones. For example, as shown for FIG. 4, user-specific tooth brushing behavior file 400 comprises headers or metadata including session_start_time 402a (*e.g.*, a start time a brushing session), brushing_duration 402b (*e.g.*, a brushing duration of the brushing session), brush_score 402c (*e.g.*, a brush score defining how well a user brushed (a scale of 0-100 with 100 being a perfect score) during brushing session), coverage_percentage 402d (*e.g.*, a teeth coverage percentage duration of the brushing session), pressure_duration 402e (*e.g.*, a pressure duration of the brushing session), pressure_event_count 402f (*e.g.*, a number of instances of pressure vents during the brushing session), brushtime_zone_top_left 402g (*e.g.*, a brush time of the top left zone during the brushing session), brushtime_zone_top_center 402h (*e.g.*, a brush time of the top center zone during the brushing session), brushtime_zone_top_right 402i *(e.g.,* a brush time of the top right zone during the brushing session), brushtime_zone_bottom_left 402j (*e.g.*, a brush time of the bottom left zone during the brushing session), brushtime_zone_bottom_center402k (*e.g.*, a brush time of the bottom center zone during the brushing session), and brushtime_zone_bottom_right 4021 (*e.g.*, a brush time of the bottom center zone during the brushing session).

The headers or metadata of user-specific tooth brushing behavior file 400 are further described in Table 1 below. Table 1 illustrates an example prompt engineered for execution by an AI agent program (*e.g*., AI agent program 122), in accordance with various embodiments disclosed herein. In particular, Table 1 includes a pre-engineered prompt that defines descriptions of headers or metadata, including each of the headers or metadata 402a-402k as shown for FIG. 4. The pre-engineered prompt of Table 1 is provided to AI agent program 122, which may provide the pre-engineered prompt to a generative AI model as described herein.

| Table 1 (pre-engineered prompt) | |
|---|---|
| Please provide executable Python code. Start by loading an oral activity file from the specified file named {{oral_activity_file}}. This file contains the following columns: | |
| | • session_start_time: Timestamp indicating when the user started brushing. |
| | • brushing_duration: Integer representing the duration of the brushing session in seconds. The ideal duration is over 120 seconds. |
| | • brush_score: Integer score ranging from 0 to 100, reflecting the quality of the brushing. A score of 100 is the best, while 0 is the worst. |
| | • coverage_percentage: Float indicating the percentage of teeth surfaces brushed during the session (between 0 and 1). |
| | • pressure_duration: Integer indicating the duration (in seconds) of excess pressure applied. The ideal duration is less than 3 seconds. |
| | • pressure_event_count: Integer representing the number of instances of excessive pressure applied during the session. The ideal count is less than 3. |
| | • brushtime_zone_top_left: Integer indicating the time (in seconds) spent brushing the top left mouth zone. The ideal duration is over 20 seconds. |
| | • brushtime_zone_top_center: Integer indicating the time (in seconds) spent brushing the top center mouth zone. The ideal duration is over 20 seconds. |
| | • brushtime_zone_top_right: Integer indicating the time (in seconds) spent brushing the top right mouth zone. The ideal duration is over 20 seconds. |
| | • brushtime_zone_bottom_left: Integer indicating the time (in seconds) spent brushing the bottom left mouth zone. The ideal duration is over 20 seconds. |
| | • brushtime_zone_bottom_center: Integer indicating the time (in seconds) spent brushing the bottom center mouth zone. The ideal duration is over 20 seconds. |
| | • brushtime_zone_bottom_right: Integer indicating the time (in seconds) spent brushing the bottom right mouth zone. The ideal duration is over 20 seconds. |
| This data file provides valuable insights into the user's brushing history and can answer questions about brushing habits, such as duration, coverage, and frequency. Each row corresponds to a single brushing session. | |
| Steps to Follow: | |
| | 1. Information Collection: If additional information is needed, use code to gather it from the oral activity file. Once you have sufficient information, proceed with your task using your language skills. |
| | 2. Task Execution: Use code to perform necessary tasks and efficiently output the results. When suitable, utilize function calls instead of writing code directly. For instance, if a user requests to adjust the toothbrush mode, use predefined functions. |
| | Available Functions: |
| adjust_brush_mode: To change the toothbrush brushing mode. If this function is required, respond with the following function call: | |
| | { |
| | "name": "adjust_brush_mode", |
| | "parameters": { |
| | "brush_mode": <brush_mode_setting> |
| | } |
| | } |
| | Guidelines for Code Execution: |
| | • Solve tasks step by step. If a plan is not provided, clearly explain your approach. |
| | • Distinguish between steps that require code execution and those that involve your language skills. |
| | • Indicate the script type in the code block. The code will be executed directly without modifications; therefore, do not suggest incomplete code that requires user intervention. |
| | • Monitor the execution results returned. If an error occurs, provide a complete new version of the code to resolve it rather than partial changes. If the error cannot be fixed, analyze the issue, reassess your assumptions, gather additional information, and consider alternative approaches. |
| | • Carefully verify your final answer, including any verifiable evidence if possible. |
| | Conclusion: |
| Reply with 'TERMINATE' after all tasks are completed and results are analyzed. Do not respond with 'TERMINATE' until the provided code has been executed and the result has been evaluated. | |

In the above referenced example, with respect to user-specific tooth brushing behavior file 400, the AI agent program can invoke a generative AI model that associates, based on the headers or metadata, the descriptions to the user-specific tooth brushing metrics. The pre-engineered prompt also includes instructions for executing executable code, for creating the content, and for formatting and transmitting the content. In such an example, the generative AI model could input the pre-engineered prompt (*e.g*., the pre-engineered prompt of Table 1). The AI agent program 122, could then, could then access the user-specific tooth brushing behavior file 400 and output at least one of the executable code or the content. The executable code or the content could be based on the output of the generative AI model. For example, the above example, the executable code or the content may comprise output defined by the descriptions of the pre-engineered prompt, *e.g*., including the descriptions defining the headers or metadata as shown for Table 1 and as included for the data in user-specific tooth brushing behavior file 400.

With further reference to FIG. 3, at block 340, method 300 comprises transmitting, by the one or more processors, the content to a computing device (*e.g*., computing device 102c and/or computing device 102c1) of the user for output by the computing device regarding the oral activity. For example, the output can comprise coaching guidance for the user.

In another example aspect, the computing device may comprise an oral care implement (*e.g.*, an electronic toothbrush). In such aspects, the content generated or otherwise determined based on execution of the pre-engineered prompt comprises computing instructions configured to change one or more settings of the oral care implement (*e.g*., the electronic toothbrush). The one or more settings may comprise settings for changing the speed of an electronic toothbrush to address the user's oral activity. For example, the computing instructions may be sent to the oral care implement and/or its computing device (*e.g*., computing device 102c), which may comprise a command to the computing device to change a brush setting of an oral care device, *e.g*., faster to slower or vice versa. The command may be transmitted to the oral care implement and/or computing device over a network, or by an app on the device itself. The command may be formatted or implemented in a format as instructed by the pre-engineered prompt, including, for example, the JavaScript Object Notation (JSON) format as described and illustrated for the "adjust_brush_mode" function of Table 1.

FIG. 5A illustrates an example user interface 502 as rendered on a display screen 500 of a computing device 102c1 of a user and depicting an example query 504 comprising natural language regarding a request visualize under brushing zones of the user (*e.g*., as identifiable by the user's user-specific tooth brushing behavior file as shown for FIG. 4 or otherwise the user's oral activity file), in accordance with various embodiments disclosed herein. For example, as shown in the example of FIG. 5A, user interface 502 may be implemented or rendered via an application (app) executing on user computing device 102c1. User interface 502 may be implemented or rendered via a native app executing on user computing device 102c1. Computing device 102c1 comprises one type of computing device, which may be used as described for computing device 102c for FIG. 1 herein. In the example of FIG. 5A, user computing device 102c1 is a computer device of a user (*e.g.*, user 101) as described for FIG. 1, *e.g*., where 102c1 is illustrated as an APPLE iPhone that implements the APPLE iOS operating system and that has display screen 500. Computing device 102c1 may execute one or more native applications (apps) on its operating system, including, for example, an app comprising computing instructions inputting and sending queries, invoking AI agent program and/or AI agent routers (*e.g*., as server 110), and receiving and/or displaying content on a user interface and/or otherwise display screen 500 of computing device 102c11. Such native apps may be implemented or coded (*e.g.*, as computing instructions) in a computing language (*e.g.*, SWIFT) executable by the user computing device operating system (*e.g*., APPLE iOS) by the processor of user computing device 102c1. The aforementioned implementation(s) or otherwise disclosure applies equally herein for FIGs. 5C, 6A, and 6C as further described herein.

Additionally, or alternatively, user interface 502 may be implemented or rendered via a web interface, such as via a web browser application, *e.g*., Safari and/or Google Chrome app(s), or other such web browser or the like. The aforementioned implementation(s) or otherwise disclosure applies equally herein for FIGs. 5C, 6A, 6C, and 6D as further described herein.

As shown for FIG. 5A, user interface 502 comprises a text input for entering a query 504, which in the example of FIG. 5A comprise a text input "Can you show me where I under brush in a picture?" The input may be provided via a voice prompt or a virtual keyboard of computing device 102c1.

An AI agent program (*e.g*., AI agent program 122) can dynamically determine, using the natural language (*e.g.*, including its related tokens), an executable code (*e.g.*, executable code 520 as shown for FIG. 5B). to address the oral activity. In the example of FIG. 5A, the AI agent program provides an initial response 506 describing the content (*e.g*., a bar chart as described and shown for FIG. 5C) that the AI agent will dynamically determine (*e.g*., identify or generate).

FIG. 5B illustrates an example of executable code 520 comprising computing instructions as dynamically determined by the AI agent using the natural language of the query of FIG. 5A as input and programmed to address the oral activity as identified for FIG. 5A, in accordance with various embodiments disclosed herein. In one aspect, the executable code 520 is generated by the AI agent. Additionally, or alternatively, in order aspects the executable code 520 can be identified by the AI agent, where the executable code 520 can be preexisting or otherwise stored on server 110.

In the example of FIG. 5B, executable code 520 comprises approximately 30 lines of python code that are dynamically determined (*e.g*., generated or identified) by an AI agent program (*e.g.*, AI agent program 122), where the executable code is configured, when executed, to read the user-specific tooth brushing behavior file 400 (*e.g.*, "session_user.csv") and dynamically generate content, e.g., a bar chart with statistics of various brush zones for the user as shown for FIG. 5C.

FIG. 5C illustrates an example content for responding to the query of FIG. 5A, as dynamically generated based on the executable code 520 as described for FIG. 5B, and as transmitted to and displayed on the computing device 102c1 of the user of FIG. 5A, in accordance with various embodiments disclosed herein. FIG. 5C depicts an example user interface 530 as rendered on display screen 500 of computing device 102c1.

In the example of FIG. 5C, bar chart 532 is content that is dynamically generated based on accessing user-specific tooth brushing behavior file 400. The content is generated based on execution of executable code 520 and comprises a bar chart 532 graphically depicting the user's brushing time for each of the plurality of mouth zones. Bar chart 532 displayed on display screen 500 illustrates a bar chart depicting the average brushing time for each mouth time. In particular, bar chart 532 displays bars for each zone (*e.g.*, as described by headers or metadata 402a-402k as shown and described for FIG. 4 and Table 1) with average brushing time (in seconds) 534 on the y-axis and mouth zone 536 on x-axis. Further, bar chart 532 graphically depicts line 538 representing an ideal duration (*e.g*., 20 seconds) for brushing across each of the plurality of mouth zones. While 20 seconds is depicted in the example of FIG. 5C, it should be understood that that different and/or additional durations are contemplated.

FIG. 5D illustrates a further example user interface 542 as rendered on a display screen 500 of a computing device 102c1 of the user of FIG. 5A depicting an example query 544 comprising natural language regarding a request to change the brush setting, in accordance with various embodiments disclosed herein. In the example of FIG. 5D, the user's query causes the AI agent program to dynamically determine (in this case, dynamically identify) preexisting executable code 546, which when executed causes the brush setting of the user's oral care implement (e.g., an electronic toothbrush) to change a brush mode to a sensitive setting. In some aspects, the preexisting executable code 546 may invoke the "adjust_brush_mode" function as defined in Table 1 herein. Further, in the example of FIG. 5D, the preexisting executable code need not be generated, but instead identified and executed based on the user's query.

FIG. 6A illustrates an example user interface 602 as rendered on a display screen 500 of a computing device of a user and depicting an example query 604 comprising natural language regarding a request to improve brushing (*e.g*., as identifiable by the user's user-specific tooth brushing behavior file as shown for FIG. 4 or otherwise the user's oral activity file), in accordance with various embodiments disclosed herein. FIG. 6A depicts an example user interface 602 as rendered on display screen 500 of computing device 102c1.

As shown for FIG. 6A, user interface 602 comprises a text input for entering a query 604, which in the example of FIG. 6A comprises a text input "Show me how I can improve my brushing?" The input may be provided via a voice prompt or a virtual keyboard of computing device 102c1.

An AI agent program (*e.g*., AI agent program 122) can dynamically determine, using the natural language (*e.g.*, including its related tokens), an executable code (*e.g.*, executable code 620 as shown for FIG. 6B) to address the oral activity. In the example of FIG. 6A, the AI agent program provides an initial response 606 describing the content (*e.g*., text or audio recommendations as described for FIG. 6C and/or video as described for FIG. 6D) that the AI agent will dynamically generate.

FIG. 6B illustrates an example of executable code 620 comprising computing instructions as dynamically determined by the AI agent using the natural language of the query of FIG. 6A as input and programmed to address the oral activity as identified for FIG. 6A, in accordance with various embodiments disclosed herein. In one aspect, the executable code 620 is generated by the AI agent. Additionally, or alternatively, in order aspects the executable code 620 can be identified by the AI agent, where the executable code 620 can be preexisting or otherwise stored on server 110.

In the example of FIG. 6B, executable code 620 comprises approximately 45 lines of python code that are dynamically determined (*e.g*., generated or identified) by an AI agent program (*e.g.*, AI agent program 122), where the executable code is configured, when executed, to read the user-specific tooth brushing behavior file 400 (*e.g.*, "session_user.csv") and dynamically generate content, *e.g*., text or audio recommendations as described for FIG. 6C and/or video as described for FIG. 6D.

FIG. 6C illustrates an example content for responding to the query of FIG. 6A, as dynamically generated based on the executable code as described for FIG. 6B, and as transmitted to and displayed on the computing device of the user of FIG. 6A, in accordance with various embodiments disclosed herein.

For example, FIG. 6C depicts text-based content 630 and text-based content 632 as rendered on display screen 500 of computing device 102c1. In the example of FIG. 6C, text-based content 630 and text-based content 632 is generated and displayed to the user (*e.g*., user 101) on display screen 500 of computing device computing device 102c1. Text-based content 630 comprises analysis of user-specific tooth brushing behavior file 400 and provides output regarding the user's specific brushing zones and/or brush behavior.

Text-based content 632 includes general tips to guide the user in improving brushing for the various zones.

It should be understood that additional and/or different content may be generated and displayed. For example, generated content may comprise user-specific coaching content comprising at least one of: textual output, audible output, or video output created to improve the user's user-specific tooth brushing behavior for one or more of the plurality of mouth zones.

In some aspects, a reward can be displayed (not shown) to the user when the user achieves an oral activity goal. Additionally, or alternatively, the user (or a second user, *e.g.,* a child) may get an award, e.g., graphical award, such as a smiley face if content suggests that brushing is correct compared to a reference brushing behavior of the user or second user.

FIG. 6D illustrates a further example content for responding to the query of FIG. 6A, as dynamically generated based on the executable code as described for FIG. 6B, and as transmitted to and displayed on the computing device of the user of FIG. 6A, in accordance with various embodiments disclosed herein. FIG. 6D depicts an example user interface 640 as rendered on display screen 500 of computing device 102c1.

In the example of FIG. 6D comprises an example of dynamically generated content (*e.g*., a video) that includes a video of a dentist or avatar (*e.g*. avatar 642) providing brushing advice or guidance specific to the user, which may include the guidance or advice as shown and described for FIG. 6C or elsewhere herein. As shown for FIG. 6D, such video content may comprise controlled lip synchronization, expression(s), and/or hand motion(s) as the video of the dentist or the avatar 642 provides the guidance in audio and video output to the user (*e.g.*, user 101) via a display of a computing device (*e.g*., computing device 102c).

Figures 7A - 7E herein relate to an example of baby care use case (e.g., a baby sleep coach). This example includes a prompt that shows how to instruct an AI agent to determine executable code regarding sleep behavior data to help guide users to achieve an acceptable baby care outcome.

FIG. 7A illustrates an example user interface 702 as rendered on a display screen 500 of a computing device of a user and depicting an example query 704 comprising natural language regarding a request regarding a baby care use case (*e.g.*, as identifiable by a user's user-specific baby care file as shown for FIG. 7C or otherwise herein), and in accordance with various embodiments disclosed herein.

FIG. 7B illustrates an example of executable code 720 comprising computing instructions as dynamically determined by an AI agent using the natural language of the query of FIG. 7A as input and programmed to address the baby care use case as identified for FIG. 7A, in accordance with various embodiments disclosed herein. In one aspect, the executable code 720 is generated by the AI agent. Additionally, or alternatively, in order aspects the executable code 720 can be identified by the AI agent, where the executable code 720 can be preexisting or otherwise stored on server 110.

FIG. 7C illustrates an example of a user-specific baby care file 730 as accessible by executable code 720 as described by FIG. 7B, in accordance with various embodiments disclosed herein. The user-specific baby care file 730 includes data defined by headers or metadata comprising date 732a, day_time 732b, naps 732c, day_time_sleep 732d, wake_window 732e, night_sleep 732f, and night_feed 732g, each related to data captured for baby care.

FIG. 7D illustrates an example of a pre-engineered prompt 740, or portion thereof, that defines descriptions of headers or metadata, including each of the headers or metadata of the user-specific baby care file as described for FIG. 7C, where the pre-engineered prompt (or portion thereof) of FIG. 7C can be provided to an AI agent program that can provide the pre-engineered prompt (or portion thereof) to a generative AI model as described herein, in accordance with various embodiments disclosed herein.

FIG. 7E illustrates an example content 752 for responding to the query of FIG. 7A, as dynamically generated based on the executable code as described for FIG. 7B and the data file and descriptions as described for FIGs. 7C and 7D, and as transmitted to and displayed on the computing device of the user of FIG. 7A, in accordance with various embodiments disclosed herein.

Figures 8A - 8E herein relate to an example of an air care use case (e.g., an air refresher). This example includes a prompt that demonstrates how to instruct an AI agent to determine executable code for an air care device and related usage data to help guide users to achieve an acceptable air care outcome.

FIG. 8A illustrates an example user interface 802 as rendered on a display screen 500 of a computing device of a user and depicting an example query 804 comprising natural language regarding a request regarding an air care use case (*e.g*., as identifiable by a user's user-specific air care file as shown for FIG. 8C or otherwise herein), and in accordance with various embodiments disclosed herein.

FIG. 8B illustrates an example of executable code 820 comprising computing instructions as dynamically determined by an AI agent using the natural language of the query of FIG. 8A as input and programmed to address the air care use case as identified for FIG. 8A, in accordance with various embodiments disclosed herein. In one aspect, the executable code 820 is generated by the AI agent. Additionally, or alternatively, in order aspects the executable code 820 can be identified by the AI agent, where the executable code 820 can be preexisting or otherwise stored on server 110.

FIG. 8C illustrates an example of a user-specific air care file 830 as accessible by executable code 820 as described by FIG. 8B, in accordance with various embodiments disclosed herein. The user-specific air care file 830 includes data defined by headers or metadata comprising date 832a, time_on 832b, time_off 832c, intensity 832d, perfume_type 832e, room_type 832f, season 832g, weather 832h, temperature 832i, and refill_level 832j, each related to data captured for air care.

FIG. 8D illustrates an example of a pre-engineered prompt 840, or portion thereof, that defines descriptions of headers or metadata, including each of the headers or metadata of the user-specific air care file as described for FIG. 8C, where the pre-engineered prompt (or portion thereof) of FIG. 8C can be provided to an AI agent program that can provide the pre-engineered prompt (or portion thereof) to a generative AI model as described herein, in accordance with various embodiments disclosed herein.

FIG. 8E illustrates an example content 852 for responding to the query of FIG. 8A, as dynamically generated based on the executable code as described for FIG. 8B and the data file and descriptions as described for FIGs. 8C and 8D, including an intermediate executable code 852int for generating content 854, and as transmitted to and displayed on the computing device of the user of FIG. 8A, in accordance with various embodiments disclosed herein.

Figures 9A - 9E herein relate to an example of a surface care use case (e.g., a mopping device). This example includes a prompt that demonstrates how to instruct an AI agent to determine executable code for a mopping device and related usage data to help guide users to achieve an acceptable floor cleaning outcome.

FIG. 9A illustrates an example user interface 902 as rendered on a display screen 500 of a computing device of a user and depicting an example query 904 comprising natural language regarding a request regarding a surface care use case (*e.g*., as identifiable by a user's user-specific surface care file as shown for FIG. 9C or otherwise herein), and in accordance with various embodiments disclosed herein.

FIG. 9B illustrates an example of executable code 920 comprising computing instructions as dynamically determined by an AI agent using the natural language of the query of FIG. 9A as input and programmed to address the surface care use case as identified for FIG. 9A, in accordance with various embodiments disclosed herein. In the example of FIG. 9B, the executable code 920 includes function call 921 at line 14, which is a predefined or existing executable code determined (e.g., identified) by the AI agent. In one aspect, the executable code 820 is generated by the AI agent. Additionally, or alternatively, in order aspects the executable code 920 can be identified by the AI agent, where the executable code 920 can be preexisting or otherwise stored on server 110.

FIG. 9C illustrates an example of a user-specific surface care file 930 as accessible by executable code 920 as described by FIG. 9B, in accordance with various embodiments disclosed herein. The user-specific surface care file 930 includes data defined by headers or metadata comprising date 932a, start_time 932b, end_time 932c, distance 932d, and pad_life 932e, each related to data captured for surface care.

FIG. 9D illustrates an example of a pre-engineered prompt 940, or portion thereof, that defines descriptions of headers or metadata, including each of the headers or metadata of the user-specific surface care file as described for FIG. 9C, where the pre-engineered prompt (or portion thereof) of FIG. 9C can be provided to an AI agent program that can provide the pre-engineered prompt (or portion thereof) to a generative AI model as described herein, in accordance with various embodiments disclosed herein.

FIG. 9E illustrates an example content 952 for responding to the query of FIG. 9A, as dynamically generated based on the executable code as described for FIG. 9B and the data file and descriptions as described for FIGs. 9C and 9D, and as transmitted to and displayed on the computing device of the user of FIG. 9A, in accordance with various embodiments disclosed herein.

Figures 10A - 10E herein relate to an example of grooming use case (e.g., a shaver or otherwise electric razor). The example demonstrates a prompt regarding instructing an AI agent to determine executable code for a grooming device and related data to help guide users to achieve an acceptable grooming outcome.

FIG. 10A illustrates an example user interface 1002 as rendered on a display screen 500 of a computing device of a user and depicting an example query 1004 comprising natural language regarding a request regarding a grooming care use case (*e.g.*, as identifiable by a user's user-specific grooming care file as shown for FIG. 10C or otherwise herein), and in accordance with various embodiments disclosed herein.

FIG. 10B illustrates an example of executable code 1020 comprising computing instructions as dynamically determined by an AI agent using the natural language of the query of FIG. 10A as input and programmed to address the grooming care use case as identified for FIG. 10A, in accordance with various embodiments disclosed herein. In one aspect, the executable code 1020 is generated by the AI agent. Additionally, or alternatively, in order aspects the executable code 1020 can be identified by the AI agent, where the executable code 1020 can be preexisting or otherwise stored on server 110.

FIG. 10C illustrates an example of a user-specific grooming care file 1030 as accessible by executable code as described by FIG. 10B, in accordance with various embodiments disclosed herein. The user-specific grooming care file 1030 includes data defined by headers or metadata comprising date 1032a, blade_age 1032b, time_shaved 1032c, average_pressure 1032d, and blade_chane 1032e, each related to data captured for grooming care.

FIG. 10D illustrates an example of a pre-engineered prompt 1040, or portion thereof, that defines descriptions of headers or metadata, including each of the headers or metadata of the user-specific grooming care file as described for FIG. 10C, where the pre-engineered prompt (or portion thereof) of FIG. 10C can be provided to an AI agent program that can provide the pre-engineered prompt (or portion thereof) to a generative AI model as described herein, in accordance with various embodiments disclosed herein.

FIG. 10E illustrates example content 1052 for responding to the query of FIG. 10A, as dynamically generated based on the executable code as described for FIG. 10B and the data file and descriptions as described for FIGs. 10C and 10D, including an intermediate executable code 1052int for generating content 1054, and as transmitted to and displayed on the computing device of the user of FIG. 10A, in accordance with various embodiments disclosed herein.

### ASPECTS OF THE DISCLOSURE

The following aspects are provided as examples in accordance with the disclosure herein and are not intended to limit the scope of the disclosure.

Aspect 1. An artificial intelligence (AI) agent system configured to dynamically generate content regarding oral activity, the AI agent system comprising: a computer memory communicatively coupled to one or more processors, an AI agent program comprising computing instructions stored on the computer memory, and wherein the AI agent program is accessible by the one or more processors; wherein one or more oral activity files are stored on the computer memory, each oral activity file comprising data related to oral activity of respective users during respective oral activity sessions, computing instructions stored on the computer memory that, when executed by the one or more processors, cause the one or more processors to: receive a query comprising natural language including natural language tokens defining oral activity of a user, dynamically determine, by the AI agent program using the natural language of the query as input, executable code programmed to address the oral activity, dynamically generate, based on execution of the executable code, content as output for responding to the query, and transmit the content to a computing device of the user for output by the computing device regarding the oral activity.

Aspect 2. The AI agent system of aspect 1, wherein the natural language is provided as text input or in audible voice input, and wherein the natural language tokens comprise text-based tokens or audio tokens.

Aspect 3. The AI agent system of any one of aspects 1-2, wherein the natural language tokens comprise units of computation.

Aspect 4. The AI agent system of any one of aspects 1-3, wherein the natural language tokens comprise bytes or patches.

Aspect 5. The AI agent system of any one of aspects 1-4, wherein the content as dynamically generated based on the execution of the executable code comprises one of: (a) a chart; (b) a graphic; (c) a textual output; and (d) audible output, and (e) video.

Aspect 6. The AI agent system of any one of aspects 1-5, wherein the computing instructions stored on the computer memory, when executed by the one or more processors, further cause the one or more processors to: prior to dynamically generating the content as output, generating intermediate data, executable code, or graphics, and wherein the content is generated based on the intermediate data, executable code, or graphics.

Aspect 7. The AI agent system of any one of aspects 1-6, wherein the computing device comprises an oral care implement, and wherein the content comprises computing instructions configured to change one or more settings of the oral care implement.

Aspect 8. The AI agent system of any one of aspects 1-7 further comprising an AI agent router executing on one or more processors, and wherein the computing instructions, when executed by the one or more processors, further cause the one or more processors to: analyze, by the AI agent router, the natural language tokens to select the AI agent program from one or more AI agent programs configured to provide output for the query, and route, by the AI agent router, the query to the AI agent program as selected.

Aspect 9. The AI agent system of any one of aspects 1-8, wherein a user-specific tooth brushing behavior file of the user that is stored on the computer memory and defines user-specific tooth brushing metrics of the user when the user brushed during respective user tooth brushing sessions, the user-specific tooth brushing metrics further defining a plurality of mouth zones, wherein the query comprises the natural language tokens related to oral activity of the user, wherein the AI agent program comprises a brushing AI agent program, and wherein the computing instructions, that when executed by the one or more processors, further cause the one or more processors to: access the user-specific tooth brushing metrics, invoke the brushing AI agent program to dynamically determine the executable code, and analyze the user-specific tooth brushing behavior file to determine at least one of: (a) brushing times of the user across the plurality of mouth zones; (b) brushing pressure at any one or more of the plurality of mouth zones; (c) brushing movement patterns at any one or more of the plurality of mouth zones; and/or (d) brushing dwell times at any one or more of the plurality of mouth zones.

Aspect 10. The AI agent system of aspect 9, wherein the user-specific tooth brushing behavior file comprises headers or metadata identifying each of the plurality of mouth zones, wherein a pre-engineered prompt defines descriptions of the headers or metadata, wherein the AI agent program invokes a generative AI model that associates, based on the headers or metadata, the descriptions to the user-specific tooth brushing metrics, wherein the generative AI model inputs the pre-engineered prompt and the user-specific tooth brushing behavior file and outputs at least one of the executable code or the content, and wherein the executable code or the content comprise output defined by the descriptions of the pre-engineered prompt.

Aspect 11. The AI agent system of aspect 9, wherein the content generated based on execution of the executable code comprises a chart graphically depicting the user's brushing time for each of the plurality of mouth zones.

Aspect 12. The AI agent system of aspect 11, wherein the chart graphically depicts a line representing an ideal duration for brushing across each of the plurality of mouth zones.

Aspect 13. The AI agent system of aspect 9, wherein the content comprises user-specific coaching content comprising at least one of: textual output, audible output, or video output created to improve the user's user-specific tooth brushing behavior for one or more of the plurality of mouth zones.

Aspect 14. The AI agent system of any one of aspects 1-13, wherein the AI agent program comprises or is configured to access a generative AI model for dynamically generating or determining at least one of the executable code or the content.

Aspect 15. The AI agent system of aspect 14, wherein an AI agent program provides as input a pre-engineered prompt to the generative AI model, and wherein the generative AI model generates or determines at least one of the executable code or the content based on the pre-engineered prompt.

Aspect 16. The AI agent system of any one of aspects 1-15, wherein the query comprises one of: (a) a user query of the user; or (b) a system query invoked by the one or more processors.

Aspect 17. The AI agent system of aspect 16, wherein the system query is a predefined query stored in the computer memory.

Aspect 18. The AI agent system of aspect 17, wherein the system query is linked to a graphic selectable by the user from a graphic user interface (GUI) displayed on a display screen, and wherein when the graphic is selected, the one or more processors invoke the system query.

Aspect 19. The AI agent system of aspect 17, wherein the system query is invoked periodically.

Aspect 20. The AI agent system of any one of aspects 1-19, wherein the computing instructions stored on the computer memory, when executed by the one or more processors, further cause the one or more processors to: receive a video depicting brushing activity during a brushing session and comprising multimedia tokens, wherein the query comprising the natural language includes a query about the video, analyze, by the executable code, the multimedia tokens to address the oral activity, wherein the content as output for responding to query is based at least in part on the brushing activity depicted in the video.

Aspect 21. The AI agent system of aspect 20, wherein the video includes image frames of the user, and wherein the video is captured by a second user.

Aspect 22. The AI agent system of any one of aspects 1-21, wherein a reward is displayed to the user when the user achieves an oral activity goal.

Aspect 23. The AI agent system of any one of aspects 1-22, wherein dynamically determining the executable code comprises one of: (a) dynamically identifying preexisting executable code stored on the computer memory and programmed to address a specific query; or (b) dynamically generating new executable code created to address the oral activity.

Aspect 24. An artificial intelligence (AI) agent method for dynamically generating content regarding oral activity, the AI agent method comprising: receiving, by one or more processors, a query comprising natural language including natural language tokens defining oral activity of a user, dynamically determining, by an AI agent program executing on the one or more processors and using the natural language of the query as input, executable code programmed to address the oral activity, wherein the AI agent program comprises computing instructions stored on a computer memory, and wherein one or more oral activity files are stored on the computer memory, each oral activity file comprising data related to oral activity of respective users during respective oral activity sessions, dynamically generating, based on execution of the executable code by the one or more processors, content as output for responding to the query, and transmitting, by the one or more processors, the content to a computing device of the user for output by the computing device regarding the oral activity.

Aspect 25. The AI agent method of aspect 24, wherein the natural language is provided as text input or in audible voice input, and wherein the natural language tokens comprise text-based tokens or audio tokens.

Aspect 26. The AI agent method of any one of aspects 24-25, wherein the natural language tokens comprise units of computation.

Aspect 27. The AI agent method of any one of aspects 24-26, wherein the natural language tokens comprise bytes or patches.

Aspect 28. The AI agent method of any one of aspects 24-27, wherein the content as dynamically generated based on the execution of the executable code comprises one of: (a) a chart; (b) a graphic; (c) a textual output; and (d) audible output, and (e) video.

Aspect 29. The AI agent method of any one of aspects 24-28 further comprising: prior to dynamically generating the content as output, generating intermediate data, executable code, or graphics, and wherein the content is generated based on the intermediate data, executable code, or graphics.

Aspect 30. The AI agent method of any one of aspects 24-29, wherein the computing device comprises an oral care implement, and wherein the content comprises computing instructions configured to change one or more settings of the oral care implement.

Aspect 31. The AI agent method of any one of aspects 24-30 further comprising: analyzing, by an AI agent router executing on the one or more processors, the natural language tokens to select the AI agent program from one or more AI agent programs configured to provide output for the query, routing, by the AI agent router, the query to the AI agent program as selected.

Aspect 32. The AI agent method of any one of aspects 24-31, wherein a user-specific tooth brushing behavior file of the user that is stored on the computer memory and defines user-specific tooth brushing metrics of the user when the user brushed during respective user tooth brushing sessions, the user-specific tooth brushing metrics further defining a plurality of mouth zones, wherein the query comprises the natural language tokens related to oral activity of the user, wherein the AI agent program comprises a brushing AI agent program, and wherein the AI agent method further comprises: accessing the user-specific tooth brushing metrics, invoking the brushing AI agent program to dynamically determine the executable code, and analyzing the user-specific tooth brushing behavior file to determine at least one of: (a) brushing times of the user across the plurality of mouth zones; (b) brushing pressure at any one or more of the plurality of mouth zones; (c) brushing movement patterns at any one or more of the plurality of mouth zones; and/or (d) brushing dwell times at any one or more of the plurality of mouth zones.

Aspect 33. The AI agent method of aspect 32, wherein the user-specific tooth brushing behavior file comprises headers or metadata identifying each of the plurality of mouth zones, wherein a pre-engineered prompt defines descriptions of the headers or metadata, wherein the AI agent program invokes a generative AI model that associates, based on the headers or metadata, the descriptions to the user-specific tooth brushing metrics, wherein the generative AI model inputs the pre-engineered prompt and the user-specific tooth brushing behavior file and outputs at least one of the executable code or the content, and wherein the executable code or the content comprise output defined by the descriptions of the pre-engineered prompt.

Aspect 34. The AI agent method of aspect 32, wherein the content generated based on execution of the executable code comprises a chart graphically depicting the user's brushing time for each of the plurality of mouth zones.

Aspect 35. The AI agent method of aspect 34, wherein the chart graphically depicts a line representing an ideal duration for brushing across each of the plurality of mouth zones.

Aspect 36. The AI agent method of aspect 32, wherein the content comprises user-specific coaching content comprising at least one of: textual output, audible output, or video output created to improve the user's user-specific tooth brushing behavior for one or more of the plurality of mouth zones.

Aspect 37. The AI agent method of any one of aspects 24-36, wherein the AI agent program comprises or is configured to access a generative AI model for dynamically generating or determining at least one of the executable code or the content.

Aspect 38. The AI agent method of aspect 37, wherein an AI agent program provides as input a pre-engineered prompt to the generative AI model, and wherein the generative AI model generates or determines at least one of the executable code or the content based on the pre-engineered prompt.

Aspect 39. The AI agent method of any one of aspects 24-38, wherein the query comprises one of: (a) a user query of the user; or (b) a system query invoked by the one or more processors.

Aspect 40. The AI agent method of aspect 39, wherein the system query is a predefined query stored in the computer memory.

Aspect 41. The AI agent method of aspect 40, wherein the system query is linked to a graphic selectable by the user from a graphic user interface (GUI) displayed on a display screen, and wherein when the graphic is selected, the one or more processors invoke the system query.

Aspect 42. The AI agent method of aspect 41, wherein the system query is invoked periodically.

Aspect 43. The AI agent method of any one of aspects 24-42 further comprising: receiving a video depicting brushing activity during a brushing session and comprising multimedia tokens, wherein the query comprising the natural language includes a query about the video, analyzing, by the executable code, the multimedia tokens to address the oral activity, wherein the content as output for responding to query is based at least in part on the brushing activity depicted in the video.

Aspect 44. The AI agent method of aspect 43, wherein the video includes image frames of the user, and wherein the video is captured by a second user.

Aspect 45. The AI agent method of any one of aspects 24-44, wherein a reward is displayed to the user when the user achieves an oral activity goal.

Aspect 46. The AI agent method of any one of aspects 24-45, wherein dynamically determining the executable code comprises one of: (a) dynamically identifying preexisting executable code stored on the computer memory and programmed to address a specific query; or (b) dynamically generating new executable code created to address the oral activity.

Aspect 47. A tangible, non-transitory computer-readable medium storing instructions for dynamically generating content regarding oral activity, that when executed by one or more processors cause the one or more processors to: receive, by one or more processors, a query comprising natural language including natural language tokens defining oral activity of a user, dynamically determine, by an AI agent program executing on the one or more processors and using the natural language of the query as input, executable code programmed to address the oral activity, wherein the AI agent program comprises computing instructions stored on a computer memory, and wherein one or more oral activity files are stored on the computer memory, each oral activity file comprising data related to oral activity of respective users during respective oral activity sessions, dynamically generate, based on execution of the executable code by the one or more processors, content as output for responding to the query, and transmit, by the one or more processors, the content to a computing device of the user for output by the computing device regarding the oral activity.

### ADDITIONAL ASPECTS OF THE DISCLOSURE

The following additional aspects are provided as examples in accordance with the disclosure herein and are not intended to limit the scope of the disclosure.

Aspect 1. An artificial intelligence (AI) agent system configured to dynamically generate content regarding personal care activity, the AI agent system comprising: a computer memory communicatively coupled to one or more processors; an AI agent program comprising computing instructions stored on the computer memory, wherein the AI agent programs are accessible by one or more processors, and wherein one or more user activity files are stored on the computer memory, each user activity file comprising data related to user activity of respective users during respective activity sessions, computing instructions stored on the computer memory that, when executed by the one or more processors, cause the one or more processors to: receive a query comprising natural language including natural language tokens defining activity of a user, dynamically determine, by the AI agent using the natural language of the query as input, executable code programmed to address the activity, dynamically generate, based on execution of the executable code, content as output for responding to the query, and transmit the content to a computing device of the user for output by the computing device regarding the activity.

Aspect 2. The AI agent system of aspect 1, wherein the natural language is provided as text input or in audible voice input, and wherein the natural language tokens comprise text-based tokens or audio tokens.

Aspect 3. The AI agent system of any one of aspects 1-2, wherein the natural language tokens comprise units of computation.

Aspect 4. The AI agent system of any one of aspects 1-3, wherein the natural language tokens comprise bytes or patches.

Aspect 5. The AI agent system of any one of aspects 1-4, wherein the content as dynamically generated based on the execution of the executable code comprises one of: (a) a chart; (b) a graphic; (c) a textual output; and (d) audible output, and (e) video.

Aspect 6. The AI agent system of any one of aspects 1-5, wherein the computing instructions stored on the computer memory, when executed by the one or more processors, further cause the one or more processors to: prior to dynamically generating the content as output, generating intermediate data, executable code, or graphics, and wherein the content is generated based on the intermediate data, executable code, or graphics.

Aspect 7. The AI agent system of any one of aspects 1-6, wherein the computing device comprises one of: (a) an oral care implement; (b) a grooming implement; (c) a hair care device; (d) a surface care device; or (e) a sleep care device, and wherein the content comprises computing instructions configured to change one or more settings of the computing device.

Aspect 8. The AI agent system of any one of aspects 1-7 further comprising an AI agent router executing on one or more processors, and wherein the computing instructions, when executed by the one or more processors, further cause the one or more processors to: analyze, by the AI agent router, the natural language tokens to select the AI agent program from one or more AI agent programs configured to provide output for the query, and route, by the AI agent router, the query to the AI agent program as selected.

Aspect 9. The AI agent system of any one of aspects 1-8, wherein a user-specific tooth brushing behavior file of the user that is stored on the computer memory and defines user-specific tooth brushing metrics of the user when the user brushed during respective user tooth brushing sessions, the user-specific tooth brushing metrics further defining a plurality of mouth zones, wherein the query comprises the natural language tokens related to user activity of the user, wherein the AI agent program comprises a brushing AI agent program, and wherein the computing instructions, that when executed by the one or more processors, further cause the one or more processors to: access the user-specific tooth brushing metrics, invoke the brushing AI agent program to dynamically determine the executable code, and analyze the user-specific tooth brushing behavior file to determine at least one of: (a) brushing times of the user across the plurality of mouth zones; (b) brushing pressure at any one or more of the plurality of mouth zones; (c) brushing movement patterns at any one or more of the plurality of mouth zones; and/or (d) brushing dwell times at any one or more of the plurality of mouth zones.

Aspect 10. The AI agent system of aspect 9, wherein the user-specific tooth brushing behavior file comprises headers or metadata identifying each of the plurality of mouth zones, wherein a pre-engineered prompt defines descriptions of the headers or metadata, wherein the AI agent program invokes a generative AI model that associates, based on the headers or metadata, the descriptions to the user-specific tooth brushing metrics, wherein the generative AI model inputs the pre-engineered prompt and the user-specific tooth brushing behavior file and outputs at least one of the executable code or the content, and wherein the executable code or the content comprise output defined by the descriptions of the pre-engineered prompt.

Aspect 11. The AI agent system of aspect 9, wherein the content generated based on execution of the executable code comprises a chart graphically depicting the user's brushing time for each of the plurality of mouth zones.

Aspect 12. The AI agent system of aspect 11, wherein the chart graphically depicts a line representing an ideal duration for brushing across each of the plurality of mouth zones.

Aspect 13. The AI agent system of aspect 9, wherein the content comprises user-specific coaching content comprising at least one of: textual output, audible output, or video output created to improve the user's user-specific tooth brushing behavior for one or more of the plurality of mouth zones.

Aspect 14. The AI agent system of any one of aspects 1-13, wherein the AI agent program comprises or is configured to access a generative AI model for dynamically generating or determining at least one of the executable code or the content.

Aspect 15. The AI agent system of aspect 14, wherein an AI agent program provides as input a pre-engineered prompt to the generative AI model, and wherein the generative AI model generates or determines at least one of the executable code or the content based on the pre-engineered prompt.

Aspect 16. The AI agent system of any one of aspects 1-15, wherein the query comprises one of: (a) a user query of the user; or (b) a system query invoked by the one or more processors.

Aspect 17. The AI agent system of aspect 16, wherein the system query is a predefined query stored in the computer memory.

Aspect 18. The AI agent system of aspect 17, wherein the system query is linked to a graphic selectable by the user from a graphic user interface (GUI) displayed on a display screen, and wherein when the graphic is selected, the one or more processors invoke the system query.

Aspect 19. The AI agent system of aspect 17, wherein the system query is invoked periodically.

Aspect 20. The AI agent system of any one of aspects 1-19, wherein the computing instructions stored on the computer memory, when executed by the one or more processors, further cause the one or more processors to: receive a video depicting brushing activity during a brushing session and comprising multimedia tokens, wherein the query comprising the natural language includes a query about the video, analyze, by the executable code, the multimedia tokens to address the user activity, wherein the content as output for responding to query is based at least in part on the brushing activity depicted in the video.

Aspect 21. The AI agent system of aspect 20, wherein the video includes image frames of the user, and wherein the video is captured by a second user.

Aspect 22. The AI agent system of any one of aspects 1-21, wherein a reward is displayed to the user when the user achieves a user activity goal.

Aspect 23. The AI agent system of any one of aspects 1-22, wherein dynamically determining the executable code comprises one of: (a) dynamically identifying preexisting executable code stored on the computer memory and programmed to address a specific query; or (b) dynamically generating new executable code created to address the user activity.

Aspect 24. An artificial intelligence (AI) agent method for dynamically generating content regarding personal care activity, the AI agent method comprising: receiving, by one or more processors, a query comprising natural language including natural language tokens defining user activity of a user, dynamically determining, by an AI agent program executing on the one or more processors and using the natural language of the query as input, executable code programmed to address the user activity, wherein the AI agent program comprises computing instructions stored on a computer memory, and wherein one or more user activity files are stored on the computer memory, each user activity file comprising data related to user activity of respective users during respective user activity sessions, dynamically generating, based on execution of the executable code by the one or more processors, content as output for responding to the query, and transmitting, by the one or more processors, the content to a computing device of the user for output by the computing device regarding the user activity.

Aspect 25. The AI agent method of aspect 24, wherein the natural language is provided as text input or in audible voice input, and wherein the natural language tokens comprise text-based tokens or audio tokens.

Aspect 26. The AI agent method of any one of aspects 24-25, wherein the natural language tokens comprise units of computation.

Aspect 27. The AI agent method of any one of aspects 24-26, wherein the natural language tokens comprise bytes or patches.

Aspect 28. The AI agent method of any one of aspects 24-27, wherein the content as dynamically generated based on the execution of the executable code comprises one of: (a) a chart; (b) a graphic; (c) a textual output; and (d) audible output, and (e) video.

Aspect 29. The AI agent method of any one of aspects 24-28 further comprising: prior to dynamically generating the content as output, generating intermediate data, executable code, or graphics, and wherein the content is generated based on the intermediate data, executable code, or graphics.

Aspect 30. The AI agent method of any one of aspects 24-29, wherein the computing device comprises one of: (a) an oral care implement; (b) a grooming implement; (c) a hair care device; (d) a surface care device; or (e) a sleep care device, and wherein the content comprises computing instructions configured to change one or more settings of the computing device.

Aspect 31. The AI agent method of any one of aspects 24-30 further comprising: analyzing, by an AI agent router executing on the one or more processors, the natural language tokens to select the AI agent program from one or more AI agent programs configured to provide output for the query, routing, by the AI agent router, the query to the AI agent program as selected.

Aspect 32. The AI agent method of any one of aspects 24-31, wherein a user-specific tooth brushing behavior file of the user that is stored on the computer memory and defines user-specific tooth brushing metrics of the user when the user brushed during respective user tooth brushing sessions, the user-specific tooth brushing metrics further defining a plurality of mouth zones, wherein the query comprises the natural language tokens related to user activity of the user, wherein the AI agent program comprises a brushing AI agent program, and wherein the AI agent method further comprises: accessing the user-specific tooth brushing metrics, invoking the brushing AI agent program to dynamically determine the executable code, and analyzing the user-specific tooth brushing behavior file to determine at least one of: (a) brushing times of the user across the plurality of mouth zones; (b) brushing pressure at any one or more of the plurality of mouth zones; (c) brushing movement patterns at any one or more of the plurality of mouth zones; and/or (d) brushing dwell times at any one or more of the plurality of mouth zones.

Aspect 33. The AI agent method of aspect 32, wherein the user-specific tooth brushing behavior file comprises headers or metadata identifying each of the plurality of mouth zones, wherein a pre-engineered prompt defines descriptions of the headers or metadata, wherein the AI agent program invokes a generative AI model that associates, based on the headers or metadata, the descriptions to the user-specific tooth brushing metrics, wherein the generative AI model inputs the pre-engineered prompt and the user-specific tooth brushing behavior file and outputs at least one of the executable code or the content, and wherein the executable code or the content comprise output defined by the descriptions of the pre-engineered prompt.

Aspect 34. The AI agent method of aspect 32, wherein the content generated based on execution of the executable code comprises a chart graphically depicting the user's brushing time for each of the plurality of mouth zones.

Aspect 35. The AI agent method of aspect 34, wherein the chart graphically depicts a line representing an ideal duration for brushing across each of the plurality of mouth zones.

Aspect 36. The AI agent method of aspect 32, wherein the content comprises user-specific coaching content comprising at least one of: textual output, audible output, or video output created to improve the user's user-specific tooth brushing behavior for one or more of the plurality of mouth zones.

Aspect 37. The AI agent method of any one of aspects 24-36, wherein the AI agent program comprises or is configured to access a generative AI model for dynamically generating or determining at least one of the executable code or the content.

Aspect 38. The AI agent method of aspect 37, wherein an AI agent program provides as input a pre-engineered prompt to the generative AI model, and wherein the generative AI model generates or determines at least one of the executable code or the content based on the pre-engineered prompt.

Aspect 39. The AI agent method of any one of aspects 24-38, wherein the query comprises one of: (a) a user query of the user; or (b) a system query invoked by the one or more processors.

Aspect 40. The AI agent method of aspect 39, wherein the system query is a predefined query stored in the computer memory.

Aspect 41. The AI agent method of aspect 40, wherein the system query is linked to a graphic selectable by the user from a graphic user interface (GUI) displayed on a display screen, and wherein when the graphic is selected, the one or more processors invoke the system query.

Aspect 42. The AI agent method of aspect 41, wherein the system query is invoked periodically.

Aspect 43. The AI agent method of any one of aspects 24-42 further comprising: receiving a video depicting brushing activity during a brushing session and comprising multimedia tokens, wherein the query comprising the natural language includes a query about the video, analyzing, by the executable code, the multimedia tokens to address the user activity, wherein the content as output for responding to query is based at least in part on the brushing activity depicted in the video.

Aspect 44. The AI agent method of aspect 43, wherein the video includes image frames of the user, and wherein the video is captured by a second user.

Aspect 45. The AI agent method of any one of aspects 24-44, wherein a reward is displayed to the user when the user achieves a user activity goal.

Aspect 46. The AI agent method of any one of aspects 24-45, wherein dynamically determining the executable code comprises one of: (a) dynamically identifying preexisting executable code stored on the computer memory and programmed to address a specific query; or (b) dynamically generating new executable code created to address the user activity.

Aspect 47. A tangible, non-transitory computer-readable medium storing instructions for dynamically generating content regarding personal care activity, that when executed by one or more processors cause the one or more processors to: receive, by one or more processors, a query comprising natural language including natural language tokens defining user activity of a user, dynamically determine, by an AI agent program executing on the one or more processors and using the natural language of the query as input, executable code programmed to address the user activity, wherein the AI agent program comprises computing instructions stored on a computer memory, and wherein one or more user activity files are stored on the computer memory, each user activity file comprising data related to user activity of respective users during respective user activity sessions, dynamically generate, based on execution of the executable code by the one or more processors, content as output for responding to the query, and transmit, by the one or more processors, the content to a computing device of the user for output by the computing device regarding the user activity.

### ADDITIONAL CONSIDERATIONS

Although the disclosure herein sets forth a detailed description of numerous different embodiments, it should be understood that the legal scope of the description is defined by the words of the claims set forth at the end of this patent and equivalents. The detailed description is to be construed as exemplary only and does not describe every possible embodiment since describing every possible embodiment would be impractical. Numerous alternative embodiments may be implemented, using either current technology or technology developed after the filing date of this patent, which would still fall within the scope of the claims.

The following additional considerations apply to the foregoing discussion. Throughout this specification, plural instances may implement components, operations, or structures described as a single instance. Although individual operations of one or more methods are illustrated and described as separate operations, one or more of the individual operations may be performed concurrently, and nothing requires that the operations be performed in the order illustrated. Structures and functionality presented as separate components in example configurations may be implemented as a combined structure or component. Similarly, structures and functionality presented as a single component may be implemented as separate components. These and other variations, modifications, additions, and improvements fall within the scope of the subject matter herein.

Additionally, certain embodiments are described herein as including logic or a number of routines, subroutines, applications, or instructions. These may constitute either software (*e.g*., code embodied on a machine-readable medium or in a transmission signal) or hardware. In hardware, the routines, etc., are tangible units capable of performing certain operations and may be configured or arranged in a certain manner. In example embodiments, one or more computer systems (*e.g*., a standalone, client or server computer system) or one or more hardware modules of a computer system (*e.g.*, a processor or a group of processors) may be configured by software (*e.g.*, an application or application portion) as a hardware module that operates to perform certain operations as described herein.

The various operations of example methods described herein may be performed, at least partially, by one or more processors that are temporarily configured (*e.g*., by software) or permanently configured to perform the relevant operations. Whether temporarily or permanently configured, such processors may constitute processor-implemented modules that operate to perform one or more operations or functions. The modules referred to herein may, in some example embodiments, comprise processor-implemented modules.

Similarly, the methods or routines described herein may be at least partially processor implemented. For example, at least some of the operations of a method may be performed by one or more processors or processor-implemented hardware modules. The performance of certain of the operations may be distributed among the one or more processors, not only residing within a single machine, but deployed across a number of machines. In some example embodiments, the processor or processors may be located in a single location, while in other embodiments the processors may be distributed across a number of locations.

The performance of certain of the operations may be distributed among the one or more processors, not only residing within a single machine, but deployed across a number of machines. In some example embodiments, the one or more processors or processor-implemented modules may be located in a single geographic location (*e.g*., within a home environment, an office environment, or a server farm). In other embodiments, the one or more processors or processor-implemented modules may be distributed across a number of geographic locations.

This detailed description is to be construed as exemplary only and does not describe every possible embodiment, as describing every possible embodiment would be impractical, if not impossible. A person of ordinary skill in the art may implement numerous alternate embodiments, using either current technology or technology developed after the filing date of this application.

Those of ordinary skill in the art will recognize that a wide variety of modifications, alterations, and combinations can be made with respect to the above described embodiments without departing from the scope of the invention, and that such modifications, alterations, and combinations are to be viewed as being within the ambit of the inventive concept.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. An artificial intelligence (AI) agent system configured to dynamically generate content regarding personal care activity, the AI agent system comprising: a computer memory communicatively coupled to one or more processors; an AI agent program comprising computing instructions stored on the computer memory, wherein the AI agent programs are accessible by one or more processors, and wherein one or more user activity files are stored on the computer memory, each user activity file comprising data related to user activity of respective users during respective activity sessions, computing instructions stored on the computer memory that, when executed by the one or more processors, cause the one or more processors to: receive a query comprising natural language including natural language tokens defining activity of a user, dynamically determine, by the AI agent using the natural language of the query as input, executable code programmed to address the activity, dynamically generate, based on execution of the executable code, content as output for responding to the query, and transmit the content to a computing device of the user for output by the computing device regarding the activity.

2. The AI agent system of claim 1, wherein the natural language is provided as text input or in audible voice input, and wherein the natural language tokens comprise text-based tokens or audio tokens.

3. The AI agent system of any one of claims 1-2, wherein the natural language tokens comprise units of computation.

4. The AI agent system of any one of claims 1-3, wherein the natural language tokens comprise bytes or patches.

5. The AI agent system of any one of claims 1-4, wherein the content is dynamically generated based on the execution of the executable code comprises one of: (a) a chart; (b) a graphic; (c) a textual output; and (d) audible output, and (e) video.

6. The AI agent system of any one of claims 1-5, wherein the computing instructions stored on the computer memory, when executed by the one or more processors, further cause the one or more processors to: prior to dynamically generating the content as output, generating intermediate data, executable code, or graphics, and wherein the content is generated based on the intermediate data, executable code, or graphics.

7. The AI agent system of any one of claims 1-6, wherein the computing device comprises comprises an oral care implement, and wherein the content comprises computing instructions configured to change one or more settings of the oral care implement.

8. The AI agent system of any one of claims 1-7, further comprising an AI agent router executing on one or more processors, and wherein the computing instructions, when executed by the one or more processors, further cause the one or more processors to: analyze, by the AI agent router, the natural language tokens to select the AI agent program from one or more AI agent programs configured to provide output for the query, and route, by the AI agent router, the query to the AI agent program as selected.

9. The AI agent system of any one of claims 1-8, wherein a user-specific tooth brushing behavior file of the user that is stored on the computer memory and defines user-specific tooth brushing metrics of the user when the user brushed during respective user tooth brushing sessions, the user-specific tooth brushing metrics further defining a plurality of mouth zones, wherein the query comprises the natural language tokens related to user activity of the user, wherein the AI agent program comprises a brushing AI agent program, and wherein the computing instructions, that when executed by the one or more processors, further cause the one or more processors to: access the user-specific tooth brushing metrics, invoke the brushing AI agent program to dynamically determine the executable code, and analyze the user-specific tooth brushing behavior file to determine at least one of: (a) brushing times of the user across the plurality of mouth zones; (b) brushing pressure at any one or more of the plurality of mouth zones; (c) brushing movement patterns at any one or more of the plurality of mouth zones; and/or (d) brushing dwell times at any one or more of the plurality of mouth zones.

10. The AI agent system of claim 9, wherein the user-specific tooth brushing behavior file comprises headers or metadata identifying each of the plurality of mouth zones, wherein a pre-engineered prompt defines descriptions of the headers or metadata, wherein the AI agent program invokes a generative AI model that associates, based on the headers or metadata, the descriptions to the user-specific tooth brushing metrics, wherein the generative AI model inputs the pre-engineered prompt and the user-specific tooth brushing behavior file and outputs at least one of the executable code or the content, and wherein the executable code or the content comprise output defined by the descriptions of the pre-engineered prompt.

11. The AI agent system of claim 9, wherein the content comprises user-specific coaching content comprising at least one of: textual output, audible output, or video output created to improve the user's user-specific tooth brushing behavior for one or more of the plurality of mouth zones.

12. The AI agent system of any one of claims 1-11, wherein the AI agent program comprises or is configured to access a generative AI model for dynamically generating or determining at least one of the executable code or the content.

13. The AI agent system of claim 12, wherein an AI agent program provides as input a pre-engineered prompt to the generative AI model, and wherein the generative AI model generates or determines at least one of the executable code or the content based on the pre-engineered prompt.

14. The AI agent system of any one of claims 1-13, wherein the computing instructions stored on the computer memory, when executed by the one or more processors, further cause the one or more processors to:
receive a video depicting brushing activity during a brushing session and comprising multimedia tokens, wherein the query comprising the natural language includes a query about the video,
analyze, by the executable code, the multimedia tokens to address the user activity, wherein the content as output for responding to query is based at least in part on the brushing activity depicted in the video.

15. The AI agent system of any one of claims 1-14, wherein dynamically determining the executable code comprises one of: (a) dynamically identifying preexisting executable code stored on the computer memory and programmed to address a specific query; or (b) dynamically generating new executable code created to address the oral activity.
